# EUROPÄISCHE PATENTANMELDUNG

(11) **EP 1 693 047 A2**
(43) Veröffentlichungstag der Anmeldung: **23.08.2006**
(21) Anmeldenummer: 06002906.3
(22) Anmeldetag: 14.02.2006
(51) Int. Cl.: A61K 8/00

(54) **Kosmetische, pharmazeutische oder dermatologische Zubereitungen enthaltend Copolymerwachse**

(30) Priorität: 22.02.2005 DE 102005007980
(71) Anmelder: Clariant Produkte (Deutschland) GmbH, 65929 Frankfurt am Main (DE)
(72) Erfinder: Heinrichs, Franz-Leo, Dr., 86456 Gablingen (DE); Lukasch, Anton, DI., 86405 Meitingen (DE); Michaelis, Heike, 64295 Darmstadt (DE); Lachmann, Angela, Dr., 65779 Kelkheim-Fischbach (DE)
(74) Vertreter: Paczkowski, Marcus

(57) **Zusammenfassung**

Es werden kosmetische, pharmazeutische und dermatologische Zubereitungen beschrieben, die Copolymerwachse enthalten. Die Copolymerwachse enthalten Struktureinheiten, die abgeleitet sind von α-Olefinen mit 26 bis 60 Kohlenstoffatomen, von Maleinsäureanhydrid, Maleinsäure oder deren Salzen und gegebenenfalls von weiteren Monomeren.

## Beschreibung

Die Erfindung betrifft kosmetische, pharmazeutische und dermatologische Zubereitungen, enthaltend Copolymerwachse aus langkettigen α-Olefinen und Maleinsäuereanhydrid, Maleinsäure oder deren Salzen.

Wachse und wachsähnliche Substanzen bestimmen hauptsächlich die Konsistenz von vielen Kosmetik-Produkten. Wachse werden verwendet, um Härte und Festigkeit von kosmetischen Produkten zu beeinflussen. Je mehr besonders harte und erst bei hohen Temperaturen schmelzende Wachse eingesetzt werden, umso fester wird das Produkt.

Wachse werden auch eingesetzt, um Wirksubstanzen mit Schutzhüllen zu versehen, um diese zu stabilisieren, deren Freisetzung zu verzögern, aber auch um deren Kompatibilität mit hydrophilen und lipophilen Substanzen und damit die Formulierbarkeit zu verbessern. Mit Hilfe von Wachsen kann zudem die Wasserfestigkeit von kosmetischen Produkten, beispielsweise von Sonnenschutzmittel erhöht werden.

In der Kosmetik werden häufig natürliche Wachse tierischen und pflanzlichen Ursprungs, wie Bienenwachs, Beerenwachs, Rosenwachs, Japanwachs, Chinawachs, Schellackwachs, Quittenwachs, Shea-Butter, Candelillawachs, Carnaubawachs, Lanolin (Wollfett) und Jojobaöl, Jojobawachs verwendet. Wegen einer Belastung natürlicher Wachse durch Pestizide sucht man nach alternativen, synthetisch hergestellten Wachsen, die frei von Pflanzenschutzmitteln und allergenen Stoffen sind.

In DE 27 27 329 werden Copolymere aus α-Olefinen und Maleinsäureanhydrid und in DE 30 03 797 Copolymere aus C₃₀₊-Olefinen und Maleinsäureanhydrid, sowie deren Umsetzungsprodukte mit aliphatischen Monoalkoholen beschrieben und deren Einsatz als Gleit- und Schmiermittel in der Metall und Kunststoff verarbeitenden Industrie offenbart.

In WO 2004/041220 und WO 2004/041150 werden kosmetische Zubereitungen beschrieben, insbesondere Lippenstifte und Make-ups, enthaltend neben einer flüssigen Fettphase ein semikristallines Polymer aus einem C₁₄-C₂₄-α-Olefin und einem weiteren Monomer ausgewählt aus Carbonsäureestern, vorzugsweise C₁₄₋C₂₄-Alkyl- oder C₁₁-C₁₅-Perflooroalkyl-(meth)acrylaten, und N-Alkyl(meth)-acrylamiden. Genannt werden auch Polymere aus einem C₁₄-C₂₄-α-Olefin und einem Carbonsäureanhydrid, beispielsweise Maleinsäureanhydrid. Die beschriebenen Polymere sind zumindest teilweise öllöslich und sind zur Herstellung temperaturbeständiger Sticks und Pasten ungeeignet.

Es bestand die Aufgabe, Substanzen für kosmetische, pharmazeutische und dermatologische Zubereitungen zur Verfügung zu stellen, die ähnlich gute konsistenzgebende Eigenschaften wie natürlich vorkommende Wachse aufweisen, mit wässrigen Systemen und mit Ölsystemen gut verträglich sind, ein klares visuelles Erscheinungsbild haben, leicht verarbeitbar und kompatibel mit Wirksubstanzen (z.B. Sonnenschutzfilter) sind, auf der Haut wasserabweisende Filme bilden, Temperatur- und Lagerbeständigkeit aufweisen, aber auch hautverträglich und toxikologisch unbedenklich sind.

Es wurde überraschenderweise gefunden, dass diese Aufgabe gelöst wird durch Copolymerwachse enthaltend Struktureinheiten, die sich ableiten von α-Olefinen mit mehr als 24 Kohlenstoffatomen, Maleinsäureanhydrid oder Maleinsäure oder deren Salzen und gegebenenfalls weiteren Monomeren. Es wurde zudem gefunden, dass diese Copolymerwachse aufgrund hoher Säurezahlen leicht emulgierbar sind, sich zur Herstellung von Wachsemulsionen verschiedenster Art eignen, mit Formulierungen auf wässriger Basis gut kompatibel sind, in Form von Wasser-in-Wachs Dispersionen zubereitet werden können, aber auch Öle gut verdicken, ein herausragendes Ölbindevermögen zeigen, als Gleitmittel, Dispergierhilfsmittel und Haftmittel geeignet sind und als polare synthetische Wachse als Ersatz für natürliche Wachse in kosmetischen, pharmazeutischen und dermatologischen Zubereitungen verwendet werden können. Darüber hinaus zeigen die Copolymerwachse zusätzliche wertvolle anwendungstechnische Eigenschaften. Sie haben ein weißes bis beiges Aussehen, neigen nicht zur Bildung großer Kristallite, sind weich und geschmeidig, gut verarbeitbar und gut geeignet zur stabilen Konsistenzgebung von Pasten, Cremes, Presslingen und weichen Stiften; sie sind geeignet zur Viskositätseinstellung cremiger Emulsionen oder Dispersionen, sowie hydroxysäurehaltiger und elektrolythaltiger Mittel, verbessern die Feinteiligkeit und Stabilität der Emulsionen und können zu fließfähigen Zubereitungen verarbeitet werden. Sie verbessern signifikant das Aufnahmevermögen der Pigmente in der Lipidphase und die Pigmentdispergierung, sowie die Wirkung von Effektpigmenten.

Sehr vorteilhaft für die kosmetische Anwendung ist auch das durch die Copolymerwachse bewirkte Frischegefühl auf der Haut, sowie das gute Spreitungsvermögen, die Wasserfestigkeit, Schweißbeständigkeit, Ölbeständigkeit und Haftfestigkeit der sie enthaltenden Zubereitungen. Die Migration fester Inhaltsstoffe (z.B. Pigmente) wird unterdrückt, ebenso die Tendenz einzelner Inhaltsstoffe in die Haut zu penetrieren. Damit wird eine Reduzierung der Reizwirkung von Inhaltsstoffen erreicht. Vorteilhaft ist auch eine verzögerte Freisetzung von Wirksubstanzen, sowie eine verbesserte Kompatibilität einzelner in kosmetischen, pharmazeutischen und dermatologischen Zubereitungen üblicher Komponenten.

Gegenstand der Erfindung sind kosmetische, pharmazeutische und/oder dermatologische Zubereitungen, die ein oder mehrere Copolymerwachse, enthaltend
a) eine oder mehrere Struktureinheiten -CH₂-CHR-, worin R für eine lineare oder verzweigte Alkylgruppe mit 24 bis 58 Kohlenstoffatomen steht,
b) eine oder mehrere Struktureinheiten der Formeln (I) - (IV) 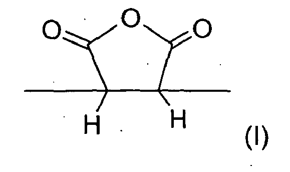 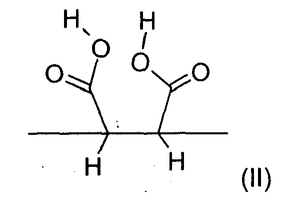 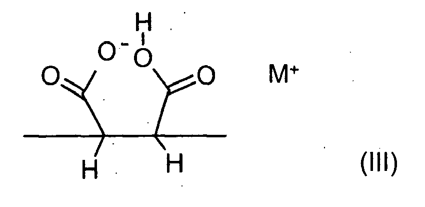 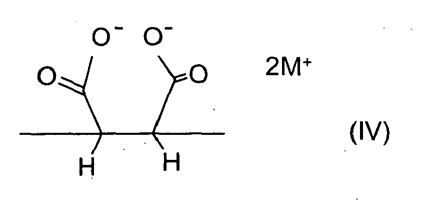 worin M⁺ für Li⁺, Na⁺, K⁺, Mg⁺⁺/2, Ca⁺⁺/2, Al⁺⁺⁺/3, NH₄⁺, Monoalkylammonium-, Dialkylammonium-, Trialkylammonium- und/oder Tetraalkylammoniumionen steht, wobei es sich bei den Alkylsubstituenten dieser Ammoniumionen jeweils unabhängig voneinander um (C₁-C₂₂)-Alkylreste oder (C₂-C₁₀)-Hydroxyalkylreste handeln kann, die auch ethoxyliert sein können und wobei die 1 bis 4 Ethylenoxid-haltigen Reste dieser Ammoniumionen jeweils unabhängig voneinander von 2 bis 10 Ethylenoxideinheiten enthalten können und den gleichen oder unterschiedliche Ethoxylierungsgrade aufweisen können und
c) gegebenenfalls eine oder mehrere Struktureinheiten der Formeln 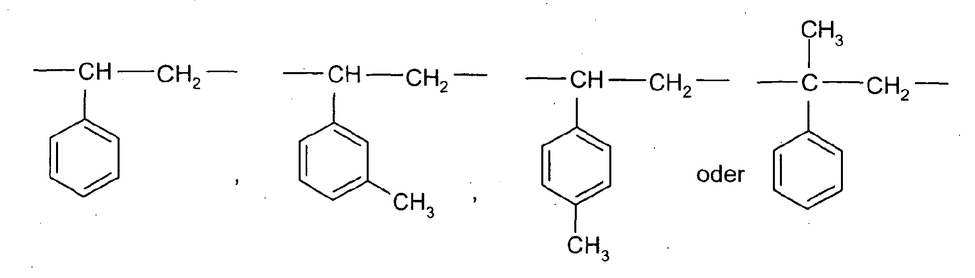 mit der Maßgabe, daß die Struktureinheiten der Komponenten a), b) und gegebenenfalls c) im Wesentlichen alternierend angeordnet sind und die Anzahl der Struktureinheiten der Komponente a), aller Struktureinheiten der Komonente b) zusammen und gegebenenfalls aller Struktureinheiten der Komponente c) zusammen jeweils im Bereich von 3 bis 60 liegt, enthalten.

In den erfindungsgemäß eingesetzten Copolymerwachsen können auch verschiedene M⁺ enthalten sein.

Die im Wesentlichen alternierende Anordnung der Struktureinheiten der Komponenten a), b) und gegebenenfalls c) bedeutet im Rahmen der vorliegenden Erfindung, dass die Struktureinheiten der jeweiligen Komponenten nur zu einem gewissen Anteil mit einer anderen Struktureinheit der gleichen Komponente verknüpft sein können. Beispielsweise sind bevorzugt 0 bis 10 mol-% und besonders bevorzugt 0 bis 5 mol-% der Struktureinheiten der Komponente b) direkt mit einer anderen Struktureinheit der Komponente b) verknüpft.

Die Struktureinheiten der Komponente a) gehen durch Polymerisation von C₂₆-C₆₀-α-Olefinen hervor.

Die Struktureinheiten der Komponente b) gehen durch Polymerisation von Maleinsäureanhydrid, die gegebenenfalls nach der Polymerisation in die Mono- oder Di-Säure oder in die entsprechenden Salze umgewandelt wird, hervor.

Bei den erfindungsgemäß eingesetzten Copolymerwachsen handelt es sich um Kammpolymere auf Basis von langkettigen Olefinen und ethylenisch ungesättigten Säuren und deren Derivaten. Die polaren und unpolaren Anteile sind im Wesentlichen alternierend angeordnet. Mit der Herstellweise der Copolymerwachse kann das Molekulargewicht, das Kristallisationsverhalten, die Viskosität, das Schmelzverhalten und die Härte beeinflußt werden.

Die Viskositäten liegen im Bereich von 100 mPas bis 4000 mPas, bevorzugt 200 mPas bis 2500 mPas, besonders bevorzugt 250 mPas bis 2000 mPas bei 140°C, die Schmelzpunkte liegen im Bereich von 30°C bis 90°C und die Härten mit Nadelpenetrationszahlen (NPZ) von 1 bis 10, bevorzugt 2 bis 6, besonders bevorzugt 3 bis 4 bei 25°C im mittleren Bereich bekannter Wachse. Das Prinzip dieser Charakterisierung gemäß der Methode ASTM D5 - und NFT 004 besteht darin, die Tiefe, ausgedrückt in Zehntel Millimeter, zu messen, bis zu der eine genormte Nadel, welche 2,5 g wiegt und in einem 47,5 g schweren Nadelträger eingesetzt ist, nach dem Aufsetzen auf das Wachs in 5 Sekunden bei einer gegebenen Temperatur eindringt.

In einer bevorzugten Ausführungsform enthalten die erfindungsgemäßen Zubereitungen Copolymerwachse, die Struktureinheiten der Komponente a) enthalten, worin R für eine lineare oder verzweigte Alkylgruppe mit 24 und/oder 26 Kohlenstoffatomen steht. Diese Struktureinheiten der Komponente a) sind abgeleitet von C_{26/28}-α-Olefinen. Es werden Copolymerwachse mit Schmelzpunkten von 30 bis 80°C erhalten, die ein gutes Spreitungsvermögen und eine gute Hautsensorik zeigen. Beim Auftragen dieser Copolymerwachse auf die Haut oder auf die Haare entstehen wasserabweisende Filme, die die Migration und das Aus- und Abwaschen von Inhaltsstoffen der kosmetischen, pharmazeutischen und dermatologischen Mittel, insbesondere von Wirksubstanzen und/oder Pigmenten, verhindern.

In einer weiteren bevorzugten Ausführungsform enthalten die erfindungsgemäßen Zubereitungen Copolymerwachse, die Struktureinheiten der Komponente a) enthalten, worin R für eine lineare oder verzweigte Alkylgruppe mit 28 bis 58 Kohlenstoffatomen steht. Diese Struktureinheiten der Komponente a) sind abgeleitet von C₃₀₊-α-Olefinen. Es werden Copolymerwachse mit Schmelzpunkten von 35 bis 75°C erhalten, die sich durch eine gute Hautsensorik auszeichnen. Zudem werden härtere Wachse erhalten, die ein sehr gutes Ölbindevermögen zeigen und mit der flüssigen Lipidphase zu festen, temperaturbeständigen Mitteln, beispielsweise Lippenstiften, verarbeitet werden können.

In einer weiteren bevorzugten Ausführungsform enthalten die erfindungsgemäßen Zubereitungen Copolymerwachse, die Struktureinheiten der Komponente a) enthalten, worin R für eine lineare oder verzweigte Alkylgruppe
- mit 28 und/oder 30 und/oder 32 Kohlenstoffatomen steht, oder
- mit 30 und/oder 32 und/oder 34 Kohlenstoffatomen steht, oder
- mit 32 und/oder 34 und/oder 36 Kohlenstoffatomen steht, oder
- mit 34 und/oder 36 und/oder 38 Kohlenstoffatomen steht, oder
- mit 36 und/oder 38 und/oder 40 Kohlenstoffatomen steht.

In einer weiteren bevorzugten Ausführungsform enthalten die erfindungsgemäßen Zubereitungen Copolymerwachse, die Struktureinheiten der Komponente a) und Struktureinheiten der Komponente b) gemäß Formel (I) enthalten.

In einer weiteren bevorzugten Ausführungsform enthalten die erfindungsgemäßen Zubereitungen Copolymerwachse, die Struktureinheiten der Komponente a) und Struktureinheiten der Komponente b) gemäß Formel (II) enthalten.

In einer weiteren bevorzugten Ausführungsform enthalten die erfindungsgemäßen Zubereitungen Copolymerwachse, die Struktureinheiten der Komponente a) und eine oder mehrere Struktureinheiten den Komponente b) ausgewählt aus Formel (III) und Formel (IV) enthalten.

In einer weiteren bevorzugten Ausführungsform der Erfindung enthalten die Copolymerwachse die Komponenten a) und b) in den molaren Verhältnissen Komponente a) : Komponente b) von 1:1-2, vorzugsweise von 1:1-1,1 und besonders bevorzugt von 1:1,05.

In einer besonders bevorzugten Ausführungsform werden Copolymerwachse eingesetzt, die sich von C_{26/28}-α-Olefinen und Maleinsäureanhydrid ableiten, die im molaren Verhältnis 1:1-2, vorzugsweise 1:1-1,1, besonders bevorzugt 1:1,05 eingesetzt wurden, wobei Copolymerwachse mit Säurezahlen von 110 bis 220, Viskositäten im Bereich von von 150 mPas bis 4000 mPas bei 140°C und Schmelzpunkten im Bereich von 75°C bis 90°C erhalten werden. Die Copolymerwachse sind weißlich-beige mit teilkristallinem Charakter.

In einer weiteren besonders bevorzugten Ausführungsform werden Copolymerwachse eingesetzt, die sich von C₃₀₊-α-Olefinen und Maleinsäureanhydrid ableiten, die im molaren Verhältnis 1:1-2, vorzugsweise 1:1-1,1, besonders bevorzugt 1:1,05 eingesetzt wurden, wobei Copolymerwachse mit Säurezahlen von 85 bis 170, Viskositäten im Bereich von 100 mPas bis 1500 mPas bei 140°C und Schmelzpunkten im Bereich von 75 bis 90°C erhalten werden. Die Copolymerwachse sind weißlich-beige mit kristallinem Charakter.

Beim Auftragen der Copolymerwachse auf die Haut oder auf die Haare entstehen wasserabweisende (hydrophobe) Filme, die die Migration und das Aus- und Abwaschen von Inhaltsstoffen der kosmetischen, pharmazeutischen und dermatologischen Zubereitungen, insbesondere der Wirksubstanzen und/oder der Pigmente, verhindern.

Die erfindungsgemäß eingesetzten Copolymere haben Molekulargewichte vorzugsweise im Bereich von 1 300 bis 50 000, besonders bevorzugt im Bereich von 5 000 bis 30 000 und insbesondere bevorzugt im Bereich von 10 000 bis 20 000.

Die Herstellung der erfindungsgemäß eingesetzten Copolymerwachse erfolgt nach den in DE 27 27 329, DE 30 03 797 und Chevron Chemical PA-18 beschriebenen Methoden.

Die oben beschriebenen Copolymerwachse sind geruchsneutral, weiß bis beige und haben ausgezeichnete Verarbeitungseigenschaften; sie sind leicht emulgierbar und daher zur Herstellung stabiler Wachsemulsionen verschiedenster Art sehr gut geeignet. Mit den Copolymerwächsen können zusammen mit Ölkomponenten streichfähige oder fließfähige Cremes, Cremeschäume oder Pasten gefertigt werden, welche bei 20°C eine Viskosität von mehr als 500 mPas aufweisen. Sie sind ausgezeichnete Konsistenzgeber, insbesondere für Formulierungen auf Öl-Basis und besitzen gute Absorptionseigenschaften, die für die Absorption von Ölen und die Dispersion von Pigmenten, Geruchsstoffen oder festen Wirkstoffen und anderen festen Zusatzstoffen genutzt werden können.

Aufgrund ihrer Härte verleihen die erfindungsgemäß eingesetzten Copolymerwachse z.B. Lippenstiften, Kajal- und Mascarastiften Stabilität auch bei höheren Temperaturen. Besonders bei Lippenstiften kann das hohe Ölbindevermögen und die hervorragende Dispergierwirkung genutzt werden. Als Binder kann das Wachs zusammen mit Lanolin, Paraffinöl, Isopropylstearat, Pigmenten und Parfüm für die Herstellung von Lidschatten, Augenbrauenstiften, Puder- und Rouge-Presslingen verwendet werden. Dabei werden die wasserabweisenden Eigenschaften, sowie die verdickende Wirkung dieser Copolymerwachse genutzt, um ein Verlaufen der Fettschminken zu unterdrücken.

Die oben beschriebenen Copolymerwachse eignen sich zur Herstellung kosmetischer, dermatologischer und pharmazeutischer Zubereitungen, besonders vorteilhaft zur Herstellung dekorativer kosmetischer Mittel, Sonnenschutzmittel, Deodorantien, Haar-pflege- und Stylingmittel, Reinigungsmittel für die Haut, insbesondere Peelings.

Bei den erfindungsgemäßen Zubereitungen kann es sich um die unterschiedlichsten kosmetischen, pharmazeutischen und dermatologischen Formulierungen handeln. Insbesondere kann es sich um Abdeckstifte, Akne-Stifte, Lippenstifte, Make-ups, Grundierungen, Gesichtspuder, Rouge, Mascara, Lidschatten, Eye-liner, Peeling-Cremes, Haarwachse, Haar-Stylingmittel, Styling-Fluids, Haarschäume, Haargele, Haarsprays, Mousse, Haaröl, Spitzenfluids, Haarkuren, Nachtcremes, Pflegecremes, Nährcremes, Parfümcremes, Bodylotions, Salben, Lippenpflegemittel, Sonnenschutzmittel, Deodorantien, Antiperspirantien, colorierte Gele in Form von Stiften, wie z.B. Mehrphasenstiften, Sticks, Pasten, Puder, Cremes, Cremeschäume, Lotionen, selbstschäumende, schaumförmige, nachschäumende oder schäumbare Emulsionen, Gele, Roll-On-Präparate oder Schäume handeln.

Eine bevorzugte Ausführungsform sind Emulsionen. Bei den Emulsionen kann es sich sowohl um Wasser-in-Öl-Emulsionen als auch um Öl-in-Wasser-Emulsionen, Mikroemulsionen, Nanoemulsionen und multiple Emulsionen handeln. Die Herstellung der Emulsionen kann in bekannter Weise, d.h. beispielsweise durch Kalt-Heiß-, Heiß/Kalt- oder PIT-Emulgierung erfolgen. Eine besonders bevorzugt Ausführungsform sind selbstschäumende, schaumförmige, nachschäumende oder schäumbare Emulsionen und Mikroemulsionen.

Mit Hilfe von Emulgatoren lassen sich eine Vielzahl von Wachzubereitungen herstellen. Die Auswahl des Emulgators ermöglicht die Herstellung von nichtionogenen und ionogene Wachsdispersionen.

Die erfindungsgemäß eingesetzten Copolymerwachse bewirken ein gutes und feinteiliges Aufnahmevermögen von Pigmenten, festen Wirkstoffen und festen Zusatzstoffen in der Ölphase und haben eine hautglättende und feuchtigkeitsspendende Wirkung.

Die erfindungsgemäßen Zubereitungen zeichnen sich durch eine besonders gute Haftung der kosmetischen Mittel auf der Haut aus und bilden hydrophobe Filme, die durch das sich bildende Hautfett kaum gelöst werden, so dass unerwünschte Farbverschiebungen der Pigmente bzw eine Migrationen der Wirkstoffe oder festen Zusatzstoffe unterbleiben.

Die erfindungsgemäßen Emulsionen enthalten mindestens
a) eines der oben beschriebenen Copolymerwachse,
b) eine Ölkomponente
c) einen Emulgator
d) optional weitere Wachse.

Die Ölkomponente kann vorteilhafterweise ausgewählt werden aus den Gruppen der Mineralöle, Mineralwachse, Öle, wie Triglyceride, Fette, Wachse und andere natürliche und synthetische Fettkörper, vorzugsweise Ester von Fettsäuren mit Alkoholen niedriger C-Zahl, z.B. mit Isopropanol, Propylenglycol oder Glycerin, oder Ester von Fettalkoholen mit Alkansäuren niedriger C-Zahl mit Fettsäuren oder Alkylbenzoate.

Eine Klasse von erfindungsgemäß bevorzugten Ölen und Fetten sind die Triglyceride von linearen oder verzweigten, gesättigten oder ungesättigten, gegebenenfalls hydroxylierten C₈-C₃₀-Fettsäuren, insbesondere pflanzliche Öle, wie Sonnenblumen-, Mais-, Soja-, Reis-, Jojoba-, Babusscu-, Kürbis-, Traubenkern-, Sesam-, Walnuss-, Aprikosen-, Orangen-, Weizenkeim-, Pfirsichkern-, Makadamia-, Avocado-, Süßmandel-, Wiesenschaumkraut-, Ricinusöl, Olivenöl, Erdnussöl, Rapsöl und Kokosnussöl, sowie synthetische Triglyceridöle, z.B. das Handelsprodukt Myritol® 318. Auch gehärtete Triglyceride sind erfindungsgemäß bevorzugt. Auch Öle tierischen Ursprungs, beispielweise Rindertalg, Perhydrosqualen oder Lanolin können eingesetzt werden.

Eine weitere Klasse von erfindungsgemäß bevorzugten Ölen und Fetten sind die Benzoesäureester von linearen oder verzweigten C₈-C₂₂-Alkanolen, z.B. die Handelsprodukte Finsolv® SB (isostearylbenzoat), Finsolv® TN (C₁₂-C₁₅-Alkylbenzoat) und Finsolv® EB (Ethylhexylbenzoat).

Eine weitere Klasse von erfindungsgemäß bevorzugten Ölen und Fetten sind die Dialkylether mit insgesamt 8 bis 36 Kohlenstoffatomen, insbesondere 12 bis 24 C-Atomen, wie z.B. Di-n-octylether (Cetiol® OE), Di-n-decylether, Di-n-nonylether, Din-undecylether, Di-n-dodecylether, Di-3-ethyldecylether, tert-Butyl-n-octylether, isoPentyl-n-octylether, 2-Methyl-pentyl-n-octylether, n-Hexyl-n-octylether, n-Octyl-n-decylether, n-Decyl-n-undecylether, n-Undecyl-n-dodecylether und n-Hexyl-n-Undecylether sowie Di-tert-butylether oder Di-iso-pentylether.

Eine weitere Klasse von erfindungsgemäß bevorzugten Ölen und Fetten sind Kohlenwasserstofföle zum Beispiel solche mit linearen oder verzweigten, gesättigten oder ungesättigten C₇-C₄₀-Kohlenstoffketten, beispielsweise Vaseline, Dodecan, Isododecan, Cholesterol, Lanolin, hydrierte Polyisobutylene, Docosane, Hexadecan, Isohexadecan, Paraffinöle, Paraffinwachse, Isoparaffinöle, z.B. die Handelsprodukte der Permethyl® -Serie, Squalan, Squalen, synthetische Kohlenwasserstoffe wie Polyisobuten und alicyclische Kohlenwasserstoffe, z.B. das Handelsprodukt 1,3-Di-(2-ethyl-hexyl)-cyclohexan (Cetiol® S), Ozokerit, Mikrowachse und Ceresin.

Ebenso in Betracht kommen verzweigte gesättigte oder ungesättigte Fettalkohole mit 6 - 30 Kohlenstoffatomen, z.B. Isostearylalkohol, sowie Guerbetalkohole.

Eine weitere Klasse von erfindungsgemäß bevorzugten Ölen und Fetten sind Hydroxycarbonsäurealkylether. Bevorzugte Hydroxycarbonsäurealkylester sind . Vollester der Glykolsäure, Milchsäure, Apfelsäure, Weinsäure oder Zitronensäure. Weitere grundsätzlich geeignete Hydroxycarbonsäuren sind Ester der β-Hydroxypropionsäure, der Tartronsäure, der D-Gluconsäure, Zuckersäure, . Schleimsäure oder Glucuronsäure. Als Alkoholkomponente dieser Ester eignen sich primäre, lineare oder verzweigte aliphatische Alkohole mit 8 bis 22 C-Atomen. Dabei sind die Ester von C₁₂-C₁₅-Fettalkoholen besonders bevorzugt. Ester dieses Typs sind im Handel erhältlich, z.B. unter dem Handelsnamen Cosmacol® der EniChem, Augusta Industriale.

Eine weitere Klasse von erfindungsgemäß bevorzugten Ölen und Fetten sind Dicarbonsäureester von linearen oder verzweigten C₂-C₁₀-Alkanolen, wie Di-n-butyladipat (Cetiol® B); Di-(2-ethylhexyl)-adipat, Di-(2-ethylhexyl)-succinat sowie Diolester wie Ethylenglycol-dioleat, Ethylenglycol-di-isotridecanoat, Propylenglycol-di(2-ethylhexanoat), Propylenglycol-di-isostearat, Propylenglycol-di-pelargonat, Butandiol-di-isostearat und Neopentylglycoldicaprylat.

Ebenso bevorzugte Öle und Fette sind symmetrische, unsymmetrische oder cyclische Ester der Kohlensäure mit Fettalkoholen, Glycerincarbonat oder Dicaprylylcarbonat (Cetiol® CC).

Eine weitere Klasse von erfindungsgemäß bevorzugten Ölen und Fetten sind die Ester von Dimeren ungesättigter C₁₂-C₂₂-Fettsäuren (Dimerfettsäuren) mit einwertigen linearen, verzweigten oder cyclischen C₂-C₁₈-Alkanolen oder mit mehrwertigen linearen oder verzweigten C₂-C₆-Alkanolen.

In einer weiteren bevorzugten Ausführungsform der Erfindung, wenn die Ölkomponente ein Silikonöl ist, liegen die erfindungsgemäßen Zubereitungen vorzugsweise in Form einer Wasser-in-Silikon Emulsion vor und enthalten Wasser, Silikon, einen oder mehrere Emulgatoren und ein oder mehrere Copolymerwachse.

An Silikonölen bzw. -wachsen stehen vorzugsweise zur Verfügung Dimethylpolysiloxane und Cyclomethicone, Polydialkylsiloxane R₃SiO(R₂SiO)ₓSiR₃, wobei R für Methyl oder Ethyl, besonders bevorzugt für Methyl, steht und x für eine Zahl von 2 bis 500 steht, beispielsweise die unter den Handelsnamen VICASIL (General Electric Company), DOW CORNING 200, DOW CORNING 225, DOW CORNING 200 (Dow Corning Corporation) erhältlichen Dimethicone, sowie die unter SilCare® Silicone 41 M65, SilCare® Silicone 41 M70, SilCare® Silicone 41 M80 (Clariant GmbH) erhältlichen Dimethicone, Stearyldimethylpolysiloxan, C₂₀-C₂₄-Alkyl-dimethylpolysiloxan, C₂₄-C₂₈-Alkyl-dimethylpolysiloxan, aber auch die unter SilCare® Silicone 41 M40, SilCare® Silicone 41 M50 (Clariant GmbH) erhältlichen Methicone, weiterhin Trimethylsiloxysilicate [(CH₂)₃SiO)_{1/2}]ₓ[SiO₂]_{y}, wobei x für eine Zahl von 1 bis 500 und y für eine Zahl von 1 bis 500 steht, Dimethiconole R₃SiO[R₂SiO]ₓSiR₂OH und HOR₂SiO[R₂SiO]ₓSiR₂OH, wobei R für Methyl oder Ethyl und x für eine Zahl bis zu 500 steht, Polyalkylarylsiloxane, beispielsweise die unter den Handelsbezeichnungen SF 1075 METHYLPHENYL FLUID (General Electric Company) und 556 COSMETIC GRADE PHENYL TRIMETHICONE FLUID (Dow Corning Corporation) erhältlichen Polymethylphenylsiloxane, Polydiarylsiloxane, Silikonharze, cyclische Silikone und amino-, fettsäure-, alkohol-, polyether-, epoxy-, fluor- und/oder alkylmodifizierte Silikonverbindungen, sowie Polyethersiloxan-Copolymere.

Erfindungsgemäße als Emulsionen vorliegende Zubereitungen enthalten einen oder mehrere Emulgatoren aus der Gruppe der nichtionischen, anionischen, kationischen oder amphoteren Emulgatoren.

Als nichtionogene Emulgatoren stehen zur Verfügung Anlagerungsprodukte von 0 bis 30 Mol Ethylenoxid und/oder 0 bis 5 Mol Propylenoxid an lineare Fettalkohole mit 6 bis 30 C-Atomen, bevorzugt 10 bis 22 C-Atomen, und ganz besonders bevorzugt 14 bis 22 C-Atomen. Einsetzbar sind beispielsweise Octanol (Caprylalkohol), Octenol, Octadienol, Decanol (Caprinalkohol), Decenol, Decadienol, Dodecanol (Laurylalkohol), Dodecadienol, Ricinolalkohol, Myristylalkohol, Cetylalkohol, Stearylalkohol, Cetearylalkohol, Oleylalkohol, Linoleylalkohol, Linolenylalkohol, Arachidylalkohol, Behenylalkohol. Einsetzbar sind auch Fettalkoholschnitte, die durch Reduktion natürlich vorkommender Triglyceride, wie Rindertalg, Palmöl, Erdnussöl, Rüböl, Baumwollsaatöl, Sojaöl, Sonnenblumenöl und Leinöl erhalten werden oder die aus Umesterungsprodukten mit entsprechenden Alkoholen aus Fettsäureestern erzeugt werden und somit ein Gemisch von unterschiedlichen Fettalkoholen darstellen. Solche Substanzen sind beispielsweise unter der Bezeichnung Stenol® , z.B. Stenol® 1618 oder Lanette® , z.B. Lanette® O und Lanette® 22 oder Lorol® , z.B. Lorol® C18 im Handel erhältlich.

Geeignet sind auch Wollwachsfette.

Besonders bevorzugt sind Cetylalkohol, Stearylalkohol, Cetearylalkohol, Arachidylalkohol und Behenylalkohol.

Eine weitere Klasse von erfindungsgemäß bevorzugten Emulgatoren sind Anlagerungsprodukte von 0 bis 30 Mol Ethylenoxid und/oder 0 bis 5 Mol Propylenoxid an lineare und/oder verzweigte, gesättigte und/oder ungesättigte Fettsäuren mit 6 bis 30 C-Atomen, bevorzugt 10 bis 22 Kohlenstoffatomen. Zu nennen sind Isostearinsäure, wie die Handelsprodukte Emersol® 871 und Emersol® 875, Isopalmitinsäuren wie Edenor® IP95, sowie alle weiteren unter der Handelsbezeichnung Edenor® (Cognis) käuflichen Fettsäuren. Weitere typische Beispiele für solche Fettsäuren sind Capronsäure, Caprylsäure, 2-Ethylhexansäure, Caprinsäure, Laurinsäure, Isotridecansäure, Myristinsäure, Palmitinsäure, Palmitoleinsäure, Stearinsäure, Isostearinsäure, Ölsäure, Elaidinsäure, Petroselinsäure, Linolsäure, Linolensäure, Elaeosterinsäure, Arachinsäure, Gadoleinsäure, Behensäure, Erucasäure und Dimere von ungesättigten Fettsäuren, sowie deren technische Mischungen. Besonders bevorzugt sind Fettsäureschnitte aus Cocosöl oder Palmöl. Insbesondere bevorzugt ist Stearinsäure.

Üblicherweise werden die Fettsäuren mit einem basischen Mittel, z.B. NaOH, neutralisiert und beispielsweise in Form ihrer Natrium-, Kalium-, Ammonium-, Calcium-, Magnesium- und Zink-Salze verwendet.

Eine weitere Klasse von einsetzbaren Emulgatoren sind Ester von gewünschtenfalls alkylierten Zuckern mit C₆-C₃₀-Fettsäuren. Als Zucker können beliebige Mono- oder Oligosaccharide eingesetzt werden. Üblicherweise werden Monosaccharide mit 5 oder 6 Kohlenstoffatomen eingesetzt, beispielsweise Ribose, Xylose, Lyxose, Altose, Glucose, Fructose, Galactose, Arabinose, Altrose, Mannose, Gulose, Idose, Talose, sowie die Desoxyzucker Rhamnose und Fucose. Auch Zucker mit 4 Kohlenstoffatomen können eingesetzt werden, z.B. Erythrose und Threose. Erfindungsgemäß geeignete Oligosaccharide sind aus zwei bis 10 Monosaccharideinheiten zusammengesetzt, z.B. Sucrose (Saccharose), Lactose oder Trehalose. Bevorzugte Zuckerbausteine sind die Monosaccharide Glucose, Fructose, Galactose, Arabinose und das Disaccharid Sucrose. Glucose und Sucrose sind besonders bevorzugt. Die Zucker können partiell mit Methyl, Ethyl-, Propyl-, Isopropyl- oder Butylgruppen verethert sein, z.B. Methylglucosid, Ethylglucösid oder Butylglucosid. Zur Veresterung können alle C₆-C₃₀-Fettsäuren und deren Mischungen verwendet werden, die vorstehend genannt wurden. Geeignet sind prinzipiell einfach und mehrfach veresterte Zucker. Bevorzugt sind die Mono-, Sesqui- und Diester, beispielsweise Sucrosemonostearat, Sucrosedistearat, Sucrosemonococoat, Sucrosedicocoat, Methylglucosidmonostearat, Methylglucosidsesquistearat, Methylglucosidisostearat, Ethylglucosidmonolaurat, Ethylglucosiddilaurat, Ethylglucosidmonococoat, Ethylglucosiddicocoat und Butylglucosidmonococoat.

Eine weitere Klasse geeigneter Emulgatoren sind C₈-C₂₂-Alkylmono- und -oligoglycoside entsprechend der allgemeinen Formel RO-(Z)ₓ, wobei R für eine C₈-C₂₂-Alkylgruppe, Z für Zucker und x für die Anzahl der Zuckereinheiten stehen. Die erfindungsgemäß verwendbaren Alkylmono- und -oligoglycoside können lediglich einen bestimmten Alkylrest R enthalten. Besonders bevorzugt sind solche Alkylmono- und -oligoglycoside, bei denen R im Wesentlichen aus C₈- und C₁₀₋Alkylgruppen, im Wesentlichen aus C₁₂- und C₁₄-Alkylgruppen, im Wesentlichen aus C₈- bis C₁₆-Alkylgruppen oder im Wesentlichen aus C₁₂- bis C₁₆-Alkylgruppen besteht. Als Zuckerbaustein Z können beliebige Mono- oder Oligosaccharide, wie sie vorstehend genannt wurden, eingesetzt werden. Bevorzugte Zuckerbausteine sind Glucose, Fructose, Galactose, Arabinose und Sucrose, wobei Glucose besonders bevorzugt ist. Die erfindungsgemäß verwendbaren Alkylmono- und -oligoglycoside enthalten im Schnitt 1,1 - 5, bevorzugt 1,1 - 2,0 und besonders bevorzugt 1,1 - 1,8 Zuckereinheiten.

Auch die alkoxylierten Homologen der genannten Alkylmono- und -oligoglycoside können erfindungsgemäß eingesetzt werden. Geeignet sind beispielsweise Cocoylglucosid, Decylglucosid, Laurylglucosid, Cetearylglucosid und Arachidylglucosid.

Besonders bevorzugt sind neben den genannten Alkylmono- und -oligoglucosiden auch die Gemische aus Alkylmono- und -oligoglucosiden und Fettalkoholen, z.B. die im Handel erhältlichen Produkte Montanov® 68 und Montanov® 202.

Eine weitere Klasse bevorzugter Emulgatoren sind die Partialester von Propylenglycol, Glycerin und Sorbitan mit C₈-C₂₂-Fettsäuren. Zur Veresterung können alle C₈-C₂₂-Fettsäuren und deren Mischungen verwendet werden, die vorstehend bereits genannt wurden. Besonders geeignete Beispiele sind Propylenglycolmonostearat, Glycerinmonolaurat, Glycerinmonostearat, Glycerindistearat, Glycerinmonooleat, Sorbitanmonolaurat, Sorbitandilaurat, Sorbitanmonostearat, Sorbitansesquistearat, Sorbitandistearat, Sorbitanmonoisostearat, Sorbitanmonooleat, Sorbitandioleat oder die Handelsprodukte Monomuls® 90-0, Monomuls® 90-L 12 und Cutina® MD. Diese Emulgatoren können durchschnittlich bis zu 10 Ethylenoxid- und/oder Propylenoxideinheiten pro Molekül enthalten.

Eine weitere bevorzugt Klasse an Emulgatoren sind Polyglycerine der Formel HO-CH₂-CHOH-CH₂[-O-CH₂-CHOH-CH₂]ₙ-O-CH₂-CHOH-CH₂OH mit n = 0-8 und deren Ester mit linearen und verzweigten C₈-C₂₂-Fettsäuren, die in der Alkylkette funktionelle Gruppen tragen können, bevorzugt Polyglyceryl-2-dipolyhydroxystearat (Handelsprodukt Dehymuls® PGPH) und Polyglyceryl-3-diisostearat (Handelsprodukt Lameform® TGI).

Eine weitere Klasse bevorzugter Emulgatoren sind Sterole (Sterine), insbesondere Cholesterol, Lanosterol, β-Sitosterol, Stigmasterol, Campesterol und Ergosterol sowie Mykosterole. Handelsübliche Sterol-Emulgatoren werden auf der Basis von Soja- oder Rapssterolen hergestellt. Erfindungsgemäß bevorzugt ist der Einsatz von Sterolen, die 5 - 10 Ethylenoxideinheiten pro Molekül enthalten. Geeignet sind z.B. die Handelsprodukte Generel® 122, Generol® 122 E 5, Generol® 122 E 10 und Generol® RE 10.

Ebenso bevorzugt einsetzbare Emulgatoren sind Phospholipide, vor allem die Phosphatidylcholine oder Lecithine. Phospholipide sind Phosphorsäurediester, seltener -monoester, von zumeist linearen gesättigten und ungesättigten C₈-C₂₂₋Fettsäuren. Bevorzugt ist Sojalecithin.

Eine weitere Klasse bevorzugter Emulgatoren sind die Veresterungsprodukte von Milchsäure oder Glykolsäure mit linearen oder verzweigten C₈-C₂₂-Fettsäuren sowie die Natrium-, Kalium-, Ammonium-, Calcium-, Magnesium- und Zink-Salze dieser Veresterungsprodukte.

Besonders bevorzugt sind Veresterungsprodukte der allgemeinen Formel (5) 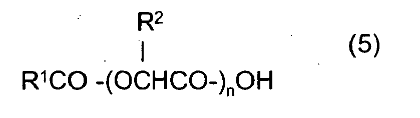 wobei R¹ einen linearen oder verzweigten gesättigten oder ungesättigten Alkylrest mit 5 bis 21 Kohlenstoffatomen und R² eine Methylgruppe oder ein Wasserstoffatom darstellen und n eine ganze Zahl von 1 - 4 ist.

Unter den Acylresten R¹CO- sind wiederum die Reste ausgewählt aus der Caproyl-, Capryloyl-, Caprinoyl-, Lauroyl-, Myristoyl-, Cetoyl-, Palmitoyl-, Stearoyl-, Isostearoyl- und der Oleyl-Gruppe bevorzugt. Besonders bevorzugt sind die Stearoyl- und die Isostearoyl-Gruppe.

Der Rest R² ist vorzugsweise Methyl.

Der Oligomerisierungsgrad n ist vorzugsweise 1 oder 2.

Insbesondere bevorzugt ist die Verbindung Natriumstearoyl-2-lactylat.

Eine weitere Klasse bevorzugt eingesetzter Emulgatoren sind Phosphorsäuremono-, -di-, und -triester von gesättigten oder ungesättigten linearen oder verzweigten Fettalkoholen mit 8 bis 30 Kohlenstoffatomen und ihre Ethylenoxidaddukte mit 1-10 Ethylenoxid-Gruppen pro Molekül. Diese Alkyl- und Alkenylphosphate sind in der allgemeinen Formel (6) dargestellt 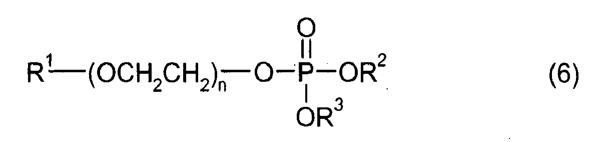 in der R¹ einen gesättigten oder ungesättigten, linearen oder verzweigten Kohlenwasserstoffrest mit 8 bis 30 Kohlenstoffatomen, R² und R³ unabhängig voneinander ein Wasserstoffatom, X oder einen Rest (CH₂CH₂O)ₙR¹, n Zahlen von 0 bis 10 und X ein Alkali- oder Erdalkalimetallkation oder ein Kation NR⁴R⁵R⁶R⁷, mit R⁴ bis R⁷ unabhängig voneinander stehend für einen C₁-C₄-Kohlenwasserstoffrest, darstellen.

Die erfindungsgemäß bevorzugten Alkyl- und Alkenylphosphate weisen als Gruppe R¹ Alkylreste mit 12- 18 Kohlenstoffatomen auf, die gesättigt oder ungesättigt sowie linear oder verzweigt sein können. Diese Gruppen R¹ sind insbesondere Lauryl, Myristyl, Cetyl, Palmityl, Stearyl, Isostearyl und Oleyl. Bevorzugte Werte für n sind entweder 0 oder Werte von 1 - 10, bevorzugt 2 - 5, besonders bevorzugt 3 - 4 (Alkyl- oder Alkenyletherphosphate). Weiterhin bevorzugt ist die Verwendung von Estergemischen aus Mono-, Di- und Triestern, wobei der Anteil an Mono- und Diester gegenüber dem Triesteranteil überwiegt. Die Verwendung von reinen Triestern kann aber ebenfalls bevorzugt sein. Geeignete Handelsprodukte stammen z.B. aus der Hostaphat® -Serie (Clariant), z.B. Hostaphat® KW 340 D, Hostaphat® KO300 N, Hostaphat® KO380 und Hostaphat® KL 340.

Eine weitere Klasse von erfindungsgemäß bevorzugt eingesetzten Emulgatoren sind Acylglutamate der Formel (7) 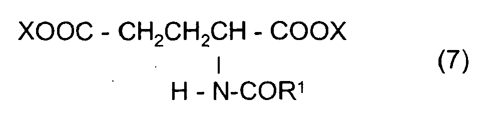 in der R¹CO für einen linearen oder verzweigten Acylrest mit 6 bis 22 Kohlenstoffatomen und 0, 1, 2 oder 3 Doppelbindungen und X für Wasserstoff, ein Alkali- oder Erdalkalimetallkation, für ein Ammonium, Alkylammonium, Alkanolammonium und/oder Glucammonium steht, beispielsweise Acylglutamate, die sich von Fettsäuren mit 6 bis 22, vorzugsweise 12 bis 18 Kohlenstoffatomen ableiten, wie beispielsweise C_{12/14}- bzw. C_{12/18}-Kokosfettsäure, Laurinsäure, Myristinsäure, Palmitinsäure und/oder Stearinsäure, insbesondere Natrium-N-cocoyl- und Natrium-N-stearoyl-L-glutamat.

Eine weitere Klasse von erfindungsgemäß bevorzugten Emulgatoren sind die Ester einer hydroxysubstituierten Di- oder Tricarbonsäure der allgemeinen Formel (8), 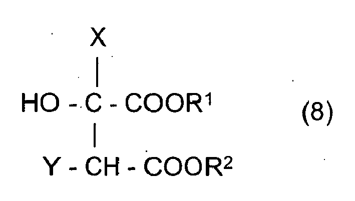 in der X H oder eine -CH₂COOR-Gruppe ist, Y H oder -OH ist unter der Bedingung, dass Y H ist, wenn X-CH₂COOR ist, R, R¹ und R² unabhängig voneinander ein Wasserstoffatom, ein Alkali- oder ein Erdalkalimetallkation, eine Ammoniumgruppe, das Kation einer ammonium-organischen Base oder einen Rest Z bedeuten, der von einer polyhydroxylierten organischen Verbindung stammt, die aus der Gruppe der veretherten (C₆-C₁₈)-Alkylsaccharide mit 1 bis 6 monomeren Saccharideinheiten und/oder der veretherten aliphatischen (C₆-C₁₆)-Hydroxyalkylpolyole mit 2 bis 16 Hydroxylresten ausgewählt ist, unter der Maßgabe, dass wenigstens eine der Gruppen R, R¹ oder R² ein Rest Z ist.

Eine weitere Klasse von erfindungsgemäß bevorzugten Emulgatoren sind die Ester des Sulfobernsteinsäuresalzes der allgemeinen Formel (9) 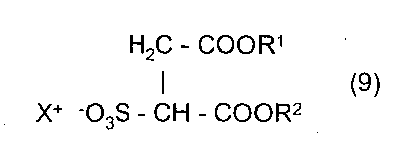 in der R¹ und R² unabhängig voneinander ein Wasserstoffatom, ein Alkali- oder Erdalkalimetallkation, eine Ammoniumgruppe, das Kation einer ammonium-organischen Base oder einen Rest Z bedeuten, der von einer polyhydroxylierten organischen Verbindung stammt, die aus der Gruppe der veretherten (C₆-C₁₈)-Alkylpolysaccharide mit 1 bis 6 monomeren Saccharideinheiten und/oder der veretherten aliphatischen (C₆-C₁₈)-Hydroxyalkylpolyole mit 2 bis 16 Hydroxylresten ausgewählt ist, unter der Maßgabe, dass wenigstens eine der Gruppen R¹ oder R² ein Rest Z ist, und X⁺ ein Alkali- oder Erdalkalimetallkation, eine Ammoniumgruppe oder das Kation einer ammonium-organischen Base bedeutet.

Eine weitere Klasse von erfindungsgemäß bevorzugten Emulgatoren sind die Sulfobernsteinsäuremono- und -dialkylester mit 8 bis 24 C-Atomen in der Alkylgruppe und Sulfobernsteinsäuremonoalkylpolyoxyester mit 8 bis 24 C-Atomen in der Alkylgruppe und 1 bis 6 Ethoxygruppen und ihre Alkali-, Erdalkalimetall- oder Ammoniumsalze.

Eine weitere Klasse von erfindungsgemäß bevorzugten Emulgatoren sind die Ester der Weinsäure und Zitronensäure mit Alkoholen, die Anlagerungsprodukte von etwa 2 bis 10 Molekülen Ethylenoxid und/oder Propylenoxid an Fettalkohole mit 8 bis 22 C-Atomen darstellen und ihre Alkali-, Erdalkalimetall- oder Ammoniumsalze.

Weitere erfindungsgemäß bevorzugte Emulgatoren sind Ethercarbonsäuren der Formel R-O-(CH₂-CH₂O)ₓ-CH₂-COOH, in der R eine lineare Alkylgruppe mit 8 bis 30 C-Atomen und x 0 oder 1 bis 10 ist, Acylsarcosinate mit einem linearen oder verzweigten Acylrest mit 6 bis 22 Kohlenstoffatomen und 0, 1, 2 oder 3 Doppelbindungen, Acyltaurate mit einem linearen oder verzweigten Acylrest mit 6 bis 22 Kohlenstoffatomen und 0, 1, 2 oder 3 Doppelbindungen sowie Acylisethionate mit einem linearen oder verzweigten Acylrest mit 6 bis 22 Kohlenstoffatomen und 0, 1, 2 oder 3 Doppelbindungen, sowie die Alkali-, Erdalkalimetall- oder Ammoniumsalze dieser Emulgatoren.

An amphoteren Emulgatoren stehen vorzugsweise zur Verfügung Alkylaminoalkylcarbonsäuren, Betaine, Sulfobetaine und Imidazolinderivate.

Vorzugsweise sind Fettalkoholethoxylate gewählt aus der Gruppe der ethoxylierten Stearylalkohole, Cetylalkohole, Cetylstearylalkohole, insbesondere Polyethylenglykol(1 3)stearylether, Polyethylenglykol(1 4)stearylether, Polyethylenglykol(15)stearylefher, Polyethylenglykol(16)stearylether, Polyethylenglykol(17)stearylether, Polyethylenglykol(18)stearylether, Polyethylenglykol(1 9)stearylether, Polyethylenglykol(20)stearytether, Polyethylenglykol(12)isostearylether, Polyethylenglykol(13)isostearylether, Polyethylenglykol(14)isostearylether, Polyethylenglykol(15)isostearylether, Polyethylenglykol(16)isostearylether, Polyethylenglykol(17)isostearylether, Polyethylenglykol(18)isostearylether, Polyethylenglykol(19)isostearylether, Polyethylenglykol(20)isostearylether, Polyethylenglykol(13)cetylether, Polyethylenglykol(14)cetylether, Polyethylenglykol(15)cetylether, Polyethylenglykol(16)cetylether, Polyethylenglykol(17)cetylether, Polyethylenglykol(18)cetylether, Polyethylenglykol(19)cetylether, Polyethylenglykol(20)cetylether, Polyethylenglykol(13)isocetylether, Polyethylenglykol(14)isocetylether, Polyethylenglykol(1 5)isocetylether, Polyethylenglykol(16)isocetylether, Polyethylenglykol(17)isocetylether, Polyethylenglykol(18)isocetylether, Polyethylenglykol(19)isocetylether, Polyethylenglykol(20)isocetylether, Polyethylenglykol(12)oleylether, Polyethylenglykol(13)oleylether; Polyethylenglykol(14)oteylether, Polyefhylenglykol(15)oleylether, Polyethylenglykol(12)laurylether, Polyethylenglykol(12)isolaurylether, Polyethylenglykol(13)cetytstearylether, Polyethylenglykol(14)cetylstearylether, Polyethylenglykol(15)cetylstearylether, Polyethylenglykol(16)cetylstearylether, Polyethylenglykol(17)cetylstearylether, Polyethylenglykol(18)cetylstearylether, Polyethylenglykol(19)cetylstearylether, Polyethylenglykol(20)cetylstea_rylether.

Vorzugsweise sind Fettsäureethoxylate gewählt aus der Gruppe der ethoxylierten Stearate, Isostearate und Oleate, insbesondere Polyethylenglycol(20)stearat, Polyethylenglykol(21)stearat, Polyethylenglykol(22)stearat, Polyethylenglykol(23)stearat, Polyethylenglykol(24)stearat, Polyethylenglykol(25)stearat, Polyethylenglykol(12)isostearat, Polyethylenglykol(13)isostearat, Polyethylenglykol(14)isostearat, Polyethylenglykol(15)isostearat, Polyethylenglykol(16)isostearat, Polyethylenglykol(17)isostearat, Polyethylenglykol(18)isostearat, Polyethylenglykol(19)isostearat, Polyethylenglykol(20)isostearat, Polyethylenglykol(21)isostearat, Polyethylenglykol(22)isostearat, Polyethylenglykol(23)isostearat, Polyethylenglykol(24)isostearat, Polyethylenglykol(25)isostearat, Polyethylenglykol(12)oleat, Polyethylenglykol(13)oleat, Polyethylenglykol(14)oleat, Polyethylenglykol(15)oleat, Polyethylenglykol(16)oleat, Polyethylenglykol(17)oleat, Polyethylenglykol(18)oleat, Polyethylenglykol(19)oleat, Polyethylenglykol(20)oleat.

Als ethoxylierte Alkylethercarbonsäure oder deren Salze kann vorteilhafterweise das Natrium-laureth (11 EO)-carboxylat verwendet werden.

Als Alkylethersulfat ist Lauryldiglycolethersulfat-Natriumsalz, als ethoxyliertes Cholesterinderivat Polyethylenglykol(30)Cholesterylether vorteilhaft. Ebenso bevorzugt ist Polyethylenglykol(25)Sojasterol.

Als ethoxylierte Triglyceride können vorteilhaft Polyethylenglykol(60)Evening Primose Glyceride verwendet werden.

Weiterhin ist es von Vorteil, die Polyethylenglykolglycerinfettsäureester aus der Gruppe Polyethylenglykol(20)glyceryllaurat, Polyethylenglykol(6)glycerylcaproat/caprinat, Polyethylenglykol(20)glyceryloleat, Polyethylenglykol(20)glycerylisostearat und Polyethylenglykol(18)glyceryloleat/cocoat, zu wählen.

Unter den Sorbitanestern eignen sich besonders Polyethylenglykol (20)sorbitanmonolaurat, Polyethylenglycol (20)sorbitanmonostearat, Polyethylenglycol (20)sorbitanmonoisostearat, Polyethylenglycol (20)sorbitanmonopalmitat, Polyethylenglykol (20)sorbitanmonooleat.

Als vorteilhafte W/O-Emulgatoren können eingesetzt werden: Fettalkohole mit 8 bis 30 Kohlenstoffatomen, Monoglycerinester gesättigter und/oder ungesättigter, verzweigter und/oder unverzweigter Alkancarbonsäuren einer Kettenlänge von 8 bis 24, insbesondere 12 bis 18 C-Atomen, Diglycerinester gesättigter und/oder ungesättigter, verzweigter und/oder unverzweigter Alkancarbonsäuren einer Kettenlänge von 8 bis 24, insbesondere 12 bis 18 C-Atomen, Monoglycerinether gesättigter und/oder ungesättigter, verzweigter und/oder unverzweigter Alkohole einer Kettenlänge von 8 bis 24, insbesondere 12 bis 18 C-Atomen, Diglycerinether gesättigter und/oder ungesättigter, verzweigter und/oder unverzweigter Alkohole einer Kettenlänge von 8 bis 24, insbesondere 12 bis 18 C-Atomen, Propylenglycolester gesättigter und/oder ungesättigter, verzweigter und/oder unverzweigter Alkancarbonsäuren einer Kettenlänge von 8 bis 24, insbesondere 12 bis 18 C-Atomen, sowie Sorbitanester gesättigter und/oder ungesättigter, verzweigter und/oder unverzweigter Alkancarbonsäuren einer Kettenlänge von 8 bis 24, insbesondere 12 bis 18 C-Atomen.

Insbesondere vorteilhafte W/O-Emulgatoren sind Glycerylmonostearat, Glycerylmonoisostearat, Glycerylmonomyristat, Glycerylmonooleat, Glycerylmonolaurat, Glycerylmonocaprylat, Glycerylmonocaprinat, Diglycerylmonostearat, Diglycerylmonoisostearat, Propylenglykolmonostearat, Propylenglykolmonoisostearat, Propylenglykolmonocaprylat, Propylenglycolmonolaurat, Sorbitanmonoisostearat, Sorbitanmonolaurat, Sorbitanmonocaprylat, Sorbitanmonoisooleat, Saccharosedistearat, Cetylalkohol, Stearylalkohol, Arachidylalkohol, Behenylalkohol, Isobehenylalkohol, Selachylalkohol, Chimylalkohol oder Polyethylenglycol(2)-stearylether.

In den erfindungsgemäßen Zubereitungen können Gemische von Verbindungen aus mehreren dieser Substanzklassen enthalten sein.
In einer weiteren bevorzugten Ausführungsform liegen die erfindungsgemäßen Zubereitungen in Form von Öl-in-Wasser-Emulsionen vor.

In einer besonders bevorzugten Ausführungsform liegen die erfindungsgemäßen Zubereitungen als Emulsionen vom Typ Öl-in-Wasser, vorzugsweise als kosmetische oder dermatologische Emulsionen vom Typ ÖI-in-Wasser, vor und enthalten bezogen auf das Gesamtgewicht der Zubereitung.
a) bis zu 95 Gew.-%, vorzugsweise 60 bis 92 Gew.-%, besonders bevorzugt 70 bis 90 Gew.-%, insbesondere bevorzugt 75 bis 85 Gew.-% einer Wasserphase,
b) bis zu 40 Gew.-%, vorzugsweise 1 bis 40 Gew.-%, besonders bevorzugt 2 bis 25 Gew.-%, insbesondere bevorzugt 5 bis 20 Gew.-% einer Ölphase,
c) bis zu 15 Gew.-%, vorzugsweise 0,5 bis 12 Gew.-%, besonders bevorzugt 1 bis 8 Gew.-%, insbesondere bevorzugt 1 bis 5 Gew.-% eines oder mehrerer Emulgatoren und
d) bis zu 5 Gew.-%, vorzugsweise 0,01 bis 5 Gew.-%, besonders bevorzugt 0,05 bis 3 Gew.-%, insbesondere bevorzugt 0,1 bis 2 Gew.-% an Copolymerwachs.

In einer weiteren besonders bevorzugten Ausführungsform liegen die erfindungsgemäßen Zubereitungen als Gelcremes vom Typ Öl-in-Wasser, vorzugsweise als kosmetische oder dermatologische Gelcremes vom Typ Öl-in-Wasser, vor und enthalten bezogen auf das Gesamtgewicht der Zubereitung
a) bis zu 95 Gew.-%, vorzugsweise 50 bis 95 Gew.-%, besonders bevorzugt 70 bis 90 Gew.-%, insbesondere bevorzugt 75 bis 85 Gew.-% einer Wasserphase,
b) bis zu 30 Gew.-%, vorzugsweise 1 bis 30 Gew.-%, besonders bevorzugt 3 bis 25 Gew.-%, insbesondere bevorzugt 5 bis 15 Gew.-% einer Ölphase,
c) bis zu 5 Gew.-%, vorzugsweise 0,5 bis 5 Gew.-%, besonders bevorzugt 0,2 bis 4 Gew.-%, insbesondere bevorzugt 0,5 bis 3 Gew.-% eines oder mehrerer Emulgatoren und
d) bis zu 5 Gew.-%, vorzugsweise 0,01 bis 5 Gew.-%, besonders bevorzugt 0,05 bis 3 Gew.-%, insbesondere bevorzugt 0,1 bis 2 Gew.-% an Copolymerwachs.

In einer weiteren bevorzugten Ausführungsform liegen die erfindungsgemäßen Zubereitungen in Form von Wasser-in-Öl-Emulsionen vor.

In einer besonders bevorzugten Ausführungsform liegen die erfindungsgemäßen Zubereitungen als Emulsionen vom Typ Wasser-in-Öl, vorzugsweise als kosmetische oder dermatologische Emulsionen vom Typ Wasser-in-Öl, vor und enthalten bezogen auf das Gesamtgewicht der Zubereitungen
a) bis zu 95 Gew.-%, vorzugsweise 40 bis 95 Gew.-%, besonders bevorzugt 50 bis 90 Gew.-%, insbesondere bevorzugt 60 bis 85 Gew.-% einer Wasserphase,
b) bis zu 60 Gew.-%, vorzugsweise 2 bis 60 Gew.-%, besonders bevorzugt 5 bis 40 Gew.-%, insbesondere bevorzugt 10 bis 30 Gew.-% einer Ölphase,
c) bis zu 20 Gew.-%, vorzugsweise 0,5 bis 20 Gew.-%, besonders bevorzugt 1 bis 15 Gew.-%, insbesondere bevorzugt 4 bis 12 Gew.-% eines oder mehrerer Emulgatoren und
d) bis zu 5 Gew.-%, vorzugsweise 0,01 bis 5 Gew.-%, besonders bevorzugt 0,05 bis 3 Gew.-%, insbesondere bevorzugt 0,1 bis 2 Gew.-% an Copolymerwachs.

In einer weiteren besonders bevorzugten Ausführungsform liegen die erfindungsgemäßen Zubereitungen als Wasser-in-Silikon-Emulsionen, vorzugsweise als kosmetische oder dermatologische Wasser-in-Silikon-Emulsionen, vor und enthalten bezogen auf das Gesamtgewicht der Zubereitung
a) bis zu 90 Gew.-%, vorzugsweise 20 bis 90 Gew.-%, besonders bevorzugt 40 bis 85 Gew.-%, insbesondere bevorzugt 60 bis 80 Gew.-% einer Wasserphase,
b) bis 80 Gew.-%, vorzugsweise 10 bis 70 Gew.-%, besonders bevorzugt 20 bis 60 Gew.-%, insbesondere bevorzugt 30 bis 50 Gew.-% an Silikonöl,
c) 0,5 bis 20 Gew.-%, vorzugsweise 1 bis 15 Gew.-%, besonders bevorzugt 3 bis 10 Gew.-% eines oder mehrerer Emulgatoren und
d) 0,01 bis 5 Gew.-%, vorzugsweise 0,05 bis 3 Gew.-%, besonders bevorzugt 0,1 bis 2 Gew.-% an Copolymerwachs.

In einer weiteren bevorzugten Ausführungsform werden die erfindungsgemäß eingesetzten Copolymerwachse in Form von mikronisierten Wachsen eingesetzt, bevorzugt in Teilchengrößen von 4 µm bis 45 µm, bevorzugt 4 µm bis 20 µm . Mikronisierte Copolymerwachse aus feinsten, runden Partikeln mit enger Korngrößenverteilung werden aus einer Wachsschmelze in einem Sprühverfahren hergestellt.

Die mikronisierten Copolymerwachse lassen sich leichter dispergieren, bewirken bessere Gleiteigenschaften der Mittel und verbessern das Hautgefühl und die Verteilbarkeit der Mittel auf der Haut und auf den Haaren. Besonders vorteilhaft können die oben beschriebenen Copolymerwachse in Peelings zum Reinigen und Pflegen der Haut eingearbeitet werden.

In einer weiteren bevorzugten Ausführungsform liegen die erfindungsgemäßen Zubereitungen in Form einer Dispersion vor und enthalten
a) ein Trägermaterial, vorzugsweise ein oder mehrere Ölkomponenten und/oder Lösungsmittel,
b) ein oder mehrere Emulgatoren und
c) ein oder mehrere der oben beschriebenen Copolymerwachse und gegebenenfalls ein oder mehrere weitere Wachse.

Wachsdispersionen, enthaltend
a) ein Trägermaterial, vorzugsweise ein oder mehrere Ölkomponenten und/oder Lösungsmittel,
b) ein oder mehrere Emulgatoren und
c) ein oder mehrere der oben beschriebenen Copolymerwachse und gegebenenfalls ein oder mehrere weitere Wachse
und worin der Wachsgehalt von 20 bis 45 Gew.-% beträgt, können auch als fließfähige Zubereitung zur Einarbeitung in kosmetische, pharmazeutische und dermatologische Mittel dienen.

Die erfindungsgemäßen Zubereitungen können neben den Copolymerwachsen bzw. mikronisierten Copolymerwachsen weitere Wachse, wie z.B. Polyethylenwachse, oxidierte Polyethylenwachse, Amidwachse, Carnaubawachse, Montanwachse, Paraffinwachse, Fischer Tropsch Wachse oder Polyvinylwachse gegebenenfalls in Kombination mit hydrophilen Wachsen, wie z.B. Cetylstearylalkohol, enthalten.

In einer weiteren bevorzugten Ausführungsform liegen die erfindungsgemäßen Zubereitungen als dekorative Mittel vor.

Eine weitere bevorzugte Ausführungsform sind kosmetische und dermatologische Stifte, beispielsweise Lippenstifte, Sonnencremestifte, Antiacne-Stifte, Augenbrauenstifte, Abdeckstifte und Deo-Stifte, enthaltend
a) eine Lipidphase aus mindestens einer Ölkomponente und mindestens einem Copolymerwachs, wie oben beschrieben,
b) optional in der Lipidphase lösliche oder dispergierbare Substanzen,
c) eine wässrige Phase,
d) optional in Wasser lösliche oder dispergierbare Substanzen,
e) optional eine oder mehrere Wirksubstanzen, und
f) mindestens einen W/O-Emulgator,
wobei der Anteil der wässrigen Phase, bezogen auf die fertige Zubereitung, 30 bis 80 Gew.-% betragen kann.

In einer weiteren bevorzugten Ausführungsform, insbesondere wenn es sich um dekorative Mittel handelt, enthalten die erfindungsgemäßen kosmetischen und dermatologischen Zubereitungen ein oder mehrere Farbmittel, vorzugsweise ausgewählt aus Farblacken, Toner und Pigmenten. Hierbei liegen sie vorzugsweise in Form von Pudern, Preßlingen, Pasten, Cremes oder Sticks vor.

In einer besonders bevorzugten Ausführungsform liegen die erfindungsgemäßen Zubereitungen in Form von Suspensionen vor und enthalten bezogen auf das Gesamtgewicht der Zubereitungen
a) 0,1 bis 10 Gew.-%, vorzugsweise 0,2 bis 6 Gew.-%, besonders bevorzugt 0,3 bis 5 Gew.-% an Copolymerwachs und
b) 0,1-30 Gew.-%, vorzugsweise 0,5 bis 15 Gew.-%, besonders bevorzugt 1,0 bis 10 Gew.-% an festen Partikeln; insbesondere ausgewählt aus der Gruppe der Farbstoffe, farbgebenden Pigmente, Effekt- und Lichtschutzpigmente, Adsorbentien und Abrassivkomponenten.

In einer weiteren besonders bevorzugten Ausführungsform liegen die erfindungsgemäßen Zubereitungen als Lidschatten auf Gelbasis vor und enthalten bezogen auf das Gesamtgewicht der Zubereitungen
a) 0,1 bis 10 Gew.-%, vorzugsweise 0,2 bis 6 Gew.-%, besonders bevorzugt 0,3 bis 5 Gew.-% an Copolymerwachs und
b) 0,1 bis 30 Gew.-%, vorzugsweise 0,5 bis 15 Gew.-%, besonders bevorzugt 1,0 bis 10 Gew.-% an Farbstoffen und/oder farbgebenden Pigmenten.

Die erfindungsgemäßen Zubereitungen können feste anorganische und organische Partikel enthalten. Für dekorative Kosmetika werden farbige und auch farblose Pigmente eingesetzt. Einige der nachfolgend genannten Pigmente dienen auch als UV-Absorber bzw. Lichtschutzpigmente.

Die Farbstoffe- und -pigmente, sowohl organische als auch anorganische Farbstoffe, können aus der entsprechenden Positivliste der Kosmetikverordnung bzw. der EG-Liste kosmetischer Färbemittel ausgewählt werden. Folgende Substanzen können beispielsweise in den erfindungsgemäßen Zubereitungen eingesetzt werden.

| Chemische oder sonstige Bezeichnung | CIN | Farbe |
|---|---|---|
| Pigment Green | 10006 | grün |
| Acid Green 1 | 10020 | grün |
| 2,4-Dinitrohydroxynaphtalin-7-sulfosäure | 10316 | gelb |
| Pigment Yellow 1 | 11680 | gelb |
| Pigment Yellow 3 | 11710 | gelb |
| Pigment Orange 1 | 11725 | orange |
| 2,4-Dihydroxyazobenzol | 11920 | orange |
| Solvent Red 3 | 12010 | rot |
| 1-(2'-Chlor-4'-nitro-1'-phenylazo)-2-hydroxynaphthalin | 12085 | rot |
| Pigment Red 3 | 12120 | rot |
| Ceresrot; Sudanrot; Fettrot G | 12150 | rot |
| Pigment Red 112 | 12370 | rot |
| Pigment Red 7 | 12420 | rot |
| Pigment Brown 1 | 12480 | braun |
| 4-(2'-Methoxy-5'-sulfosäurediethylamid-1'-phenylazo)-3-hydroxy-5"-chloro-2",4"-dimethoxy-2-naphthoesäureanilid | 12490 | rot |
| Disperse Yellow 16 | 12700 | gelb |
| 1-(4-Sulfo-1-phenylazo)-4-amino-benzol-sulfosäure | 13015 | gelb |
| 2,4-Dihydroxy-azobenzol-4'-sulfosäure | 14270 | orange |
| 2-(2,4-Dimethylphenylazo-5-sulfosäure)-1-hydroxynaphthalin-4-sulfosäure | 14700 | rot |
| 2-(4-Sulfo-1-naphthylazo)-1-naphthol-4-sulfosäure | 14720 | rot |
| 2-(6-Sulfo-2,4-xylylazo)-1-naphthol-5-sulfosäure | 14815 | rot |
| 1-(4'-Sulfophenylazo)-2-hydroxynaphthalin | 15510 | orange |
| 1-(2-Sulfosäure-4-chlor-5-carbonsäure-1-phenylazo)-2-hydroxynaphthalin | 15525 | rot |
| 1-(3-Methyl-phenylazo-4-sulfosäüre)-2-hydroxynaphthalin | 15580 | rot |
| 1-(4',(8')-Sulfosäurenaphthylazo)-2-hydroxynaphthalin | 15620 | rot |
| 2-Hydroxy-1,2'-azonaphthalin-1'-sulfosäure | 15630 | rot |
| 3-Hydroxy-4-phenylazo-2-naphthylcarbonsäure | 15800 | rot |
| 1-(2-Sulfo-4-methyl-1-phenylazo)-2-naphthylcarbonsäure | 15850 | rot |
| 1-(2-Sulfo-4-methyl-5-chlor-1 -phenylazo)-2-hydroxynaphthalin-3-carbonsäure | 15865 | rot |
| 1-(2-Sulfo-1-naphthylazo)-2-hydroxynaphthalin-3-carbonsäure | 15880 | rot |
| 1-(3-Sulfo-1-phenylazo)-2-naphthol-6-sulfosäure | 15980 | orange |
| 1-(4-Sulfo-1-phenylazo)-2-naphthol-6-sulfosäure | 15985 | gelb |
| Allura Red | 16035 | rot |
| 1-(4-Sulfo-1-naphthylazo)-2-naphthol-3,6-disulfosäure | 16185 | rot |
| Acid Orange 10 | 16230 | orange |
| 1-(4-Sulfo-1-naphthylazo)-2-naphthol-6,8-disulfosäure | 16255 | rot |
| 1-(4-Sulfo-1-naphthylazo)-2-naphthol-3,6,8,-trisulfosäure | 16290 | rot |
| 8-Amino-2-phenylazo-1-naphthol-3,6-disulfosäure | 17200 | rot |
| Acid Red 1 | 18050 | rot |
| Acid Red 155 | 18130 | rot |
| Acid Yellow 121 | 18690 | gelb |
| Acid Red 180 | 18736 | rot |
| Acid Yellow 11 | 18820 | gelb |
| Acid Yellow 17 | 18965 | gelb |
| 4-(4-Sulfo-1-phenylazo)-1-(4-sulfophenyl)-5-hydroxyphrazolon-3-carbonsäure | 19140 | gelb |
| Pigment Yellow 16 | 20040 | gelb |
| 2,6-(4'-Suffo-2",4"-dimethyl)-bis-phenylazo)1,3-dihydroxybenzol | 20170 | orange |
| Acid Black 1 | 20470 | schwarz |
| Pigment Yellow 13 | 21100 | gelb |
| Pigment Yellow 83 | 21108 | gelb |
| Solvent Yellow | 21230 | gelb |
| Acid Red 163 | 24790 | rot |
| Acid Red 73 | 27290 | rot |
| 2-[4'-(4"Sulfo-1 "-phenylazo)-7'-sulfo-1'-naphthylazo]-1-hydroxy-7-aminoaphthalin-3,6-disulfosäure | 27755 | schwarz |
| 4'-[(4"-Sulfo-1"-phenylazo)-7'-suifo-1'-naphthylazo]-1-hydroxy-8-acetyl-aminonaphthalin-3,5-disulfosäure | 28440 | schwarz |
| Direct Orange 34, 39, 44, 46, 60 | 40215 | orange |
| Food Yellow | 40800 | orange |
| trans-ß-Apo-8'-Carotinaldehyd (C₃₀) | 40820 | orange |
| trans-Apo-8'-Carotinsäure (C₃₀)-ethylester | 40825 | orange |
| Canthaxanthin | 40850 | orange |
| Acid Blue 1 | 42045 | blau |
| 2,4-Disulfo-5-hydroxy-4'-4"-bis-(diethylamino)triphenylcarbinol | 42051 | blau |
| 4-[(-4-N-Ethyl-p-sulfobenzylamino)-phenyl-(4-hydroxy-2-sulfophenyl)-(methylen)-1-(N-ethyl-N-p-sulfobenzyl)-2,5-cyclohexadienimin] | 42053 | grün |
| Acid Blue 7 | 42080 | blau |
| (N-Ethyl-p-sulfobenzyl-amino-phenyl-(2-sulfophenyl)-methylen-(N-ethyl-N-p-sulfo-benzyl)-cyclohexadienimin | 42090 | blau |
| Acid Green 9 | 42100 | grün |
| Diethyl-di-sulfobenzyl-di-4-amino-2-chlor-di-2-methylfuchsinimonium | 42170 | grün |
| Basic Violet 14 | 42510 | violett |
| Basic Violet 2 | 42520 | violett |
| 2'-Methyl-4'-(N-ethyl-N-m-sulfobenzyl)-amino-4"-(N-diethyl)-amino-2-methyl-N-ethyl-N-m-sulfobenzyl-fuchsonimmonium | 42735 | blau |
| 4'-(N-Dimethyl)-amino-4"-(N-phenyl)-aminonaphtho-N-dimethyl-fuchsonimmonium | 44045 | blau |
| 2-Hydroxy-3,6-disulfo-4,4'-bis-dimethylaminonaphtofuchsinimmonium | 44090 | grün |
| Acid red | 45100 | rot |
| 3,-(2'-Methylphenylamino)-6-(2'-methyl-4'-sulfophenylamino)-9-(2"-carboxyphenyl)-xantheniumsalz | 45190 | violett |
| Acid Red 50 | 45220 | rot |
| Phenyl-2-oxyfluoron-2-carbonsäure | 45350 | gelb |
| 4,5-Dibromfluorescein | 45370 | orange |
| 2,4,5,7-Tetrabromfluorescein | 45380 | rot |
| Solvent Dye | 45396 | orange |
| Acid Red 98 | 45405 | rot |
| 3',4',5',6'-Tetrachlor-2,4,5,7-tetrabromfluorescein | 45410 | rot |
| 4,5-Diiodfluorescein | 45425 | rot |
| 2,4,5,7-Tetraiodfluorescein | 45430 | rot |
| Chinophthalon | 47000 | gelb |
| Chinophthalon-disulfosäure | 47005 | gelb |
| Acid violet 50 | 50325 | violett |
| Acid Black 2 | 50420 | schwarz |
| Pigment Violet 23 | 51319 | violett |
| 1,2-Dioxyanthrachinon, Calcium-Aluminiumkomplex | 58000 | rot |
| 3-Oxypyren-5,8,10-sulfosäure | 59040 | grün |
| 1-Hydroxy-4-N-phenyl-aminoanthrachinon | 60724 | violett |
| 1-Hydroxy-4-(4'-methylphenylamino)-anthrachinon | 60725 | violett |
| Acid Violet 23 | 60730 | violett |
| 1,4-Di(4'-methyl-phenylamino)-anthrachinon | 61565 | grün |
| 1,4-Bis-(o-sulfo-p-toluidine)-anthrachinon | 61570 | grün |
| Acid Blue 80 | 61585 | blau |
| Acid Blue 62 | 62045 | blau |
| N,N'-Dihydro-1,2,1',2'-anthrachinonazin | 69800 | blau |
| Vat Blue 6; Pigment Blue 64 | 69825 | blau |
| Vat Orange 7 | 71105 | orange |
| Indigo | 73000 | blau |
| Indigo-disulfosäure | 73015 | blau |
| 4,4'-Dimethyl-6,6'-dichlorthioindigo | 73360 | rot |
| 5,5'-Dichlor-7,7'-dimethylthioindigo | 73385 | violett |
| Quinacridone Violet 19 | 73900 | violett |
| Pigment Red 122 | 73915 | rot |
| Pigment Blue 16 | 74100 | blau |
| Phthalocyanine | 74160 | blau |
| Direct Blue 86 | 74180 | blau |
| chlorierte Phthalocyanine | 74260 | grün |
| Natural Yellow 6,19; Natural Red 1 | 75100 | gelb |
| Bixin, Nor-Bixin | 75120 | orange |
| Lycopin | 75125 | gelb |
| trans-alpha, beta- bzw. gamma-Carotin | 75130 | orange |
| Keto- und/oder Hydroxylderivate des Carotins | 75135 | gelb |
| Guanin oder Perlglanzmittel | 75170 | weiß. |
| 1 ,7-Bis-(4-hydroxy-3-methoxyphenyl)1 ,6-heptadien-3,5-dion | 75300 | gelb |
| Komplexsalz (Na,Al,Ca) der Karminsäure | 75470 | rot |
| Chlorophyll a und b; Kupferverbindungen der Chlorophylle und Chlorophylline | 75810 | grün |
| Aluminium | 77000 | weiß |
| Tonerdehydrat | 77002 | weiß |
| wasserhaltige Aluminiumsilikate | 77004 | weiß |
| Ultramarin | 77007 | blau |
| Pigment Red 101 und 102 | 77015 | rot |
| Bariumsulfat | 77120 | weiß |
| Bismutoxychlorid und seine Gemische mit Glimmer | 77163 | weiß |
| Calciumcarbonat | 77220 | weiß |
| Calciumsulfat | 77231 | weiß |
| Kohlenstoff | 77266 | schwarz |
| Pigment Black 9 | 77267 | schwarz |
| Carbo medicinalis vegetabilis | 77268:1 | schwarz |
| Chromoxid | 77288 | grün |
| Chromoxid, wasserhaltig | 77289 | grün |
| Pigment Blue 28, Pigment Green 14 | 77346 | grün |
| Pigment Metal 2 | 77400 | braun |
| Gold | 77480 | braun |
| Eisenoxide und -hydroxide | 77489 | orange |
| Eisenoxide und -hydroxide | 77491 | rot |
| Eisenoxidhydrat | 77492 | gelb |
| Eisenoxid | 77499 | schwarz |
| Mischungen aus Eisen(II)- und Eisen(III)-hexacyanoferrat | 77510 | blau |
| Pigment White 18 | 77713 | weiß |
| Mangananimoniumdiphosphat | 77742 | violett |
| Manganphosphat; Mn₃(PO₄)₂*7H₂O | 77745 | rot |
| Silber | 77820 | weiß |
| Titandioxid und seine Gemische mit Glimmer | 77891 | weiß |
| Zinkoxid | 77947 | weiß |
| 6,7-Dimethyl-9-(1'-D-ribityl)-isoalioxazin, Lactoflavin | | gelb |
| Zuckerkulör | | braun |
| Capsanthin, Capsorubin | | orange |
| Betanin | | rot |
| Benzopyriliumsalze, Anthocyanine | | rot |
| Aluminium-, Zink-, Magnesium- und Calciumstearat | | weiß |
| Bromthymolblau | | blau |
| Bromkresolgrün | | grün |
| Acid Red 195 | | rot |

Die erfindungsgemäßen Zubereitungen können die Farbstoffe und Pigmente vorzugsweise in Mengen von 0,1 bis 30 Gew.-%, besonders bevorzugt in Mengen von 0,5 bis 15 Gew.-% und insbesondere bevorzugt in Mengen von 1,0 bis 10 Gew.-%, jeweils bezogen auf das Gesamtgewicht der Zubereitungen, enthalten.

Weiterhin bevorzugt eingesetzt werden Effektpigmente. Unter Effektpigmenten sind im Rahmen der vorliegenden Erfindung Pigmente zu verstehen, die durch ihre Brechungseigenschaften besondere optische Effekte hervorrufen. Effektpigmente verleihen der behandelten Oberfläche (Haut, Haar, Schleimhaut) Glanz- oder Glittereffekte oder können durch diffuse Lichtstreuung Hautunebenheiten und Hautfältchen optisch kaschieren. Besonders geeignete Effektpigmente sind beispielsweise Glimmerpartikel, die mit mindestens einem Metalloxid beschichtet sind. Neben Glimmer, einem Schichtsilikat, sind auch Kieselgel und andere SiO₂₋Modifikationen als Träger geeignet. Ein häufig zur Beschichtung verwendetes Metalloxid ist beispielsweise Titanoxid, dem gewünschtenfalls Eisenoxid, Cr₂O₃, Berliner Blau oder Carmin beigemischt sein kann. Ober die Größe und die Form (z.B. sphärisch, ellipsoid, abgeflacht, eben, uneben) der Pigmentpartikel sowie über die Dicke der Oxidbeschichtung können die Reflexionseigenschaften beeinflusst werden. Besonders bevorzugte Effekte sind Perlglanzpigmente, beispielsweise Fischsilber (Guanin/Hypoxanthin-Mischkristalle aus Fischschuppen) und Perlmutt (vermahlene Muschelschalen), sowie monokristalline Perlglanzpigmente wie z.B. Bismuthoxychlorid (BiOCl).

Die Effekte lassen sich sowohl über die Partikelgröße als auch über die Partikelgrößenverteilung des Pigmentensembles steuern. Geeignete Partikelgrößenverteilungen reichen z.B. von 2 - 50 µm, 5 - 25 µm, 5 - 40 µm, 5 - 60 µm, 5 - 95 µm, 5-100 µm, 10 - 60 µm, 10 - 100 µm, 10 - 125 µm, 20 - 100 µm, 20 - 150 µm, sowie < 15 µm. Eine breitere Teilchengrößenverteilung z.B. von 20 - 150 µm, ruft glitzernde Effekte hervor, während eine engere Teilchengrößenverteilung von < 15 µm für eine gleichmäßige seidige Erscheinung sorgt.

Die erfindungsgemäßen Zubereitungen enthalten Effektpigmente vorzugsweise in Mengen von 0,1 - 20 Gew.-%, besonders bevorzugt 0,5-10 und insbesondere bevorzugt 1-5 Gew.-%, jeweils bezogen auf das Gesamtgewicht der Zubereitung.

Bei den bevorzugten anorganischen Lichtschutzpigmenten handelt es sich um feindisperse oder kolloiddisperse Metalloxide und Metallsalze, beispielsweise Titanoxid, Zinkoxid, Eisenoxid, Aluminiumoxid, Ceroxid, Zirkoniumoxid, Silikate (Talk) und Bariumsulfat. Die Partikel sollten dabei einen mittleren Durchmesser von weniger als 100 nm, vorzugsweise zwischen 5 und 50 nm und besonders bevorzugt zwischen 15 und 30 nm aufweisen, so genannte Nanopigmente. Sie können eine sphärische Form aufweisen, es können jedoch auch solche Partikel zum Einsatz kommen, die eine ellipsoide oder in sonstiger Weise von der sphärischen Gestalt abweichende Form besitzen. Die Pigmente können auch oberflächenbehandelt, d.h. hydrophilisiert oder hydrophobiert vorliegen. Typische Beispiele sind gecoatete Titandioxide, wie z.B. Titandioxid T 805 (Degussa) oder Eusolex® T2000 (Merck). Als hydrophobe Coatingmittel kommen dabei vor allem Silikone und dabei speziell Trialkoxyocytylsilane oder Simethicone in Frage. Besonders bevorzugt sind Titandioxid und Zinkoxid.

Die bevorzugten anorganischen Partikelsubstanzen sind hydrophil oder amphiphil. Vorteilhafterweise können sie oberflächlich beschichtet, insbesondere oberflächlich wasserabweisend behandelt sein. Beispiele hiefür sind mit Aluminiumstearat beschichtete Titanoxid-Pigmente, mit Dimethylpolysiloxan (Dimethicone) beschichtetes Zinkoxid, mit Dimethicone beschichtetes Bornitrid und mit einem Gemisch aus Dimethylpolysiloxan und Silicagel und Aluminiumoxidhydrat beschichtetes Titanoxid, mit Octylsilanol beschichtetes Titanoxid oder sphärische Polyalkylsesquioxan-Partikel.

Die erfindungsgemäßen Zubereitungen können teilchenförmige anorganische oder organische Adsorbentien mit mittleren Partikeldurchmessern von 1-100 µm, enthalten. Die Adsorbentien sind ausgewählt aus pyrogenen Kieselsäuren, z.B. den Aerosol-Typen, Fällungskieselsäuren, Kieselgelen, Siliciumdioxid, Tonen, z.B. Bentonite oder Kaolin, Magnesiumaluminiumsilikaten, z.B. Talkum und Bornitrid, gegebenenfalls modifizierte Stärken und Stärkederivate, Cellulosepulvern, Lactoglobulinderivaten, Polymerpulvern aus Polyolefinen, Polycarbonaten, Polyurethanen, Polyamiden, Polyestern, Polystyrolen, Polyacrylaten, (Meth)acrylat- oder (Meth)acrylat-Vinyliden-Copolymeren, die vernetzt sein können, Teflon oder Silikonen, sowie Mischungen der genannten Substanzen.

Die erfindungsgemäßen Zubereitungen können Abrasivkomponenten enthalten, beispielsweise gemahlene Pflanzenteile wie Mandelkleie oder Weizenkleie, kristalline Cellulose, gehärtetes Jojobaöl, Polymerkügelchen, bevorzugt aus Polyethylen oder Polyamid-11, mit mittleren Durchmessern von 90-600 µm, und aus wirkstoffhaltigen Mikro- oder Millikapsein, die petrochemische Polymere (z.B. aus Polyamid wie Nylon-11) und/oder Biopolymere wie Gelatine, Pektin, Pflanzlichen Gummen, Alginaten und Carrageenan enthalten. Bevorzugt eingesetzt werden Mandelkleie, Weizenkleie, gehärtetes Jojobaöl und Polyethylenkügelchen.

In einer weiteren bevorzugten Ausführungsform der Erfindung handelt es sich um make-up, Augen-make-up, Mascara, eyeliner und Rouge, gekennzeichnet durch besondere Wasserfestigkeit, Farbbrillianz, Perlglanzeffekt, gute Hautsensorik und gute Verteilbarkeit der Mittel auf der Haut.

In einer weiteren bevorzugten Ausführungsform der Erfindung handelt es sich um Nagellack mit ausgezeichneten Glanzeffekten.

Eine weitere bevorzugte Ausführungsform sind Peeling-Cremes und -gele zum Reinigen und Glätten der Haut.

In einer weiteren bevorzugten Ausführungsform enthalten die erfindungsgemäßen Zubereitungen ein oder mehrere UV-Lichtschutzfilter. Diese Zubereitungen sind vorzugsweise Sonnenschutzmittel und liegen besonders bevorzugt in Form von Sprays, Sticks, Pasten, Gelen oder Lotionen vor.

Als UV-Filter kommen vorzugsweise in Betracht 4-Aminobenzoesäure; 3-(4'-Trimethylammonium)benzyliden-boran-2-on-methylsulfat; 3,3,5-Trimethyl-cyclohexylsalicylat; 2-Hydroxy-4-methoxybenzophenon; 2-Phenylbenzimidazol-5-sulfonsäure und ihre Kalium-, Natrium- und Triethanolaminsalze; 3,3'-(1,4-Phenylendimethin)-bis-(7,7-dimethyl-2-oxobicyclo[2.2.1]-heptan-1-methansulfonsäure und ihre Salze; 1-(4-tert.-Butylphenyl)-3-(4-methoxyphenyl)propan-1,3-dion, 3-(4'-Sulfo)-benzyliden-bornan-2-on und seine Salze; 2-Cyan-3,3-diphenyl-acrylsäure-(2-ethylhexylester); Polymer von N-[2(und 4)-(2-oxoborn-3-ylidenmethyl)benzyl]-acrylamid; 4-Methoxy-zimtsäure-2-ethyl-hexylester; ethoxyliertes Ethyl-4-amino-benzoat; 4-Methoxy-zimtsäureisoamylester; 2,4,6-Tris-[p-(2-ethylhexyloxycarbonyl)anilino]-1,3,5-triazin; 2-(2H-benzotriazol-2-yl)-4-methyl-6-(2-methyl-3-(1,3,3,3-tetramethyl-1-(trimethylsilyloxy)-disiloxanyl)propyl)phenol; 4,4'-[(6-[4-((1,1-dimethylethyl)-amino-carbonyl)phenylamino]-1,3,5-triazin-2,4-yl)diimino]bis-(benzoesäure-2-ethylhexylester); 3-(4'-Methylbenzyliden)-D,L-Campher; 3-Benzyliden-Campher; Sälicylsäure-2-ethylhexylester; 4-Dimethylaminobenzoesäure-2-ethylhexylester; Hydroxy-4-methoxy-benzophenon-5-sulfonsäure (Sulisobenzonum) und das Natriumsalz; und/oder 4-Isopropylbenzylsalicylat, N,N,N-Trimethyl-4-(2-oxoborn-3-ylidenemethyl) anilium methyl sulphate, Homosalate (INN) Oxybenzone (INN) 2-Phenylbenzimidazole-5-sulfonsäure und ihre Na, K, und Triethanolaminsalze, alpha-(2-Oxoborn-3-ylidene) toluol-4-sulfonsäure und ihre Salze, Octyl methoxyzimtsäure, Isopentyl-4-methoxyzimtsäure, Isoamyl p-methoxyzimtsäure, 2,4,8-Trianilino=(p-carbo-2'-ethylhexyl-1'-oxy)-1,3,5-triazine (Octyl triazone) Phenol, 2-2(2H-benzotriazol-2-yl)-4-methyl-6-(2-methyl-3-(1,3,3,3-tetramethyl-1-(trimethylsilyl)oxy)-disiloxanyl)propyl (Drometriazole Trisiloxane) benzoesäure, 4,4-((6-(((1,1-dimethylethyl)-amino)carbonyl)phenyl)amino)-1,3,5-triazine-2,4-diyl)diimino)bis-,bis(2-ethylhexyl)ester) benzoesäure, 4,4-((6-(((1,1-dimethylethyl)amino)-carbonyl)phenyl)amino)-1,3,5-triazine-2,4-diyl)diimino)bis-,bis(2-ethylhexyl)ester) 3-(4'-Methylbenzylidene)-d-1 camphor (4-Methylbenzylidene Camphor) 3-Benzylidene camphor 2-Ethylhexyl salicylat (Octyl Salicylat) 4-Dimethylamiriobenzoat von ethyl-2-hexyl (octyl dimethyl PABA), 2-Hydroxy-4-methoxybenzophenone-5-sulfonisäure (Benzophenone-5) und das Na-Salz, 2,2'-Methylen-bis-6-(2H-benzotriazol-2yl)-4-(tetramethyl-butyl)-1,1,3,3-phenol, Natriumsalz von 2-2'-bis-(1,4-phenylen)1 H-benzimidazole-4,6-disulfonsäure, (1,3,5)-Triazine-2,4-bis((4-(2-ethyl-hexyloxy)-2-hydroxy)-phenyl)-6-(4-methoxyphenyl), 2-Ethylhexyl-2-cyano-3,3-diphenyl-2-propenoat, Glyceryl octanoat Di-p-methoxy zimtsäure, p-Aminobenzoesäure und Ester, 4-tert-Butyl-4'-methoxydibenzoylmethan, 4-(2-β-glucopyranoxy)propoxy-2-hydroxybenzophenon, Octyl Salicylat, Methyl-2,5-diisopropyl zimtsäure, Cinoxat, Dihydroxy-dimethoxybenzophenon, Dinatriumsalz von 2,2'-dihydroxy-4,4'-dimethoxy-5,5'-disulfobenzophenon, Dihydroxy benzophenon, 1-3,4-Dimethoxyphenyl)-4,4-dimethyl-1,3-pentanedion, 2-Ethylhexyl dimethoxybenzylidene dioxoimidazolidin propionat, Tetrahydroxybenzophenone, Terephthalylden dicamphor sulfonsäure, 2,4,6-tris[4-2-ethylhexyloxycarbonyl)anilino]-1,3,5-triazin; Methyl bis(trimethylsiloxy)silyl isopentyl trimethoxy-zimtsäure, Amyl p-dimethylamino benzoat, 2-Ethylhexyl p-dimethylamino benzoat, Isopropyl p-methoxyzimtsäure/ Diisopropylzimtsäureester, 2-Ethylhexyl p-methoxyzimtsäure, 2-Hydroxy-4-methoxy benzophenon, 2-Hydroxy-4-methoxybenzophenon-5-sulfonsäure und das Trihydrat, 2-Hydroxy-4-methoxybenzo-phenon-5-sulfonat, Na-Salz und Phenyl-benzimidazol-sulfonsäure.

Weitere geeignete UV-Filter sind ausgewählt aus den bei Raumtemperatur festen Derivaten von Dibenzoylmethan, Zimtsäureestern, Diphenylsäureestern, Benzophenon, Capher, p-Aminobenzoesäureestern, o-Aminobenzoesäureestern, Salicylsäureestern, Benzimidazolen, 1,3,5-Triazinen, monomeren und oligomeren 4,4-Diarylbutadien-carbonsäureestern und -carbonsäureamiden, Ketotricyclo(5.2.1.0)decan, Benzalmalonsäureestern sowie beliebigen Mischungen der genannten Komponenten. Die organischen UV-Filter können öllöslich oder wasserlöslich sein. Erfindungsgemäß besonders bevorzugte öllösliche UV-Filter sind 1-Phenyl-3-(4'-Isopropylphenyl)-propan-1,3-dion, (Dimethylamino)benzoesäure-2-octylester, 4-(Dimethylamino)-benzoesäureamylester, 4-Methoxyzimtsäure-2-ethylhexylester, 4-Methoxyzimtsäure-propylester, 4-Methoxyzimtsäureisopentylester, 2-Cyano-3,3=phenylzimtsäure-2-ethylhexylester (Octocrylene), Salicylsäure-4-isopropylbenzylester, Salicylsäurehomomenthylester (3,3,5-Trimethyloctylhexylsalicylat), 2-Hydroxy-4-methoxybenzophenon, 2-Hydroxy-4-methoxy-4'-methylbenzophenon, 2,2'-Dihydroxy-4-methoxybenzophenon, 4-Methoxybenzmalonsäuredi-2-ethylhexylester, 2,4,6-Trianilino-(p-carbo-2'-ethyl-1'-hexyloxy)-1,3,5-triazin (Octyl Triazone) und Dioctyl Butamido Triazone sowie beliebige Mischungen der genannten Komponenten. Bevorzugte wasserlösliche UV-Filter sind 2-Phenylbenzimidazol-5-sulfonsäure und deren Alkali, Erdalkali, Ammonium-, Alkylammonium-, Alkanolammonium- und Glucammoniumsalze, Sulfonsäurederivate von Benzophenonen, vorzugsweise 2-Hydroxy-4-methoxybenzophenon-5-sulfonsäure und ihre Salze, Sulfonsäurederivate des 3-Benzylidencamphers, wie z.B. 4-(2-oxo-3-bornylidenmethyl)-benzolsulfonsäure und 2-Methyl-5-(2-oxo-3-bornyliden)-sulfonsäure und deren Salze.

Die erfindungsgemäßen Zubereitungen, beispielsweise die Sonnenschutzmittel, enthalten UV-Lichtschutzfilter in den Gewichtsmengen von vorzugsweise 0,1 bis 10 %, besonders bevorzugt von 0,5 bis 8 % und insbesondere bevorzugt von 1 bis 5%, bezogen auf die fertigen Zubereitungen.

Die Sonnenschutzmittel können als UV-Lichtschutzfilter aber z.B. auch die unter der oben aufgeführten Gruppe der Pigmente genannten anorganischen UV-Absorber und Lichtschutzpigmente enthalten. Dies sind in den erfindungsgemäßen Zubereitungen vorzugsweise in Mengen von 0,1 bis 30 Gew.-%, besonders bevorzugt in Mengen von 0,5 bis 15 Gew.-% und insbesondere bevorzugt in Mengen von 1,0 bis 10 Gew.-%, jeweils bezogen auf das Gesamtgewicht der Zubereitungen, enthalten.

In einer weiteren bevorzugten Ausführungsform enthalten die erfindungsgemäßen Zubereitungen, beispielsweise die Sonnenschutzmittel, ein oder mehrere Antioxidantien.

Vorteilhaft werden die Antioxidantien gewählt aus der Gruppe bestehend aus Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. (α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, y-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfon, Penta-, Hexa-, Heptathioninsulfoximin) in sehr geringen verträglichen Dosierungen, ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoehärzes, Rutinsäure und deren Derivate, α-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z.B. ZnO, ZnSO₄) Selen und dessen Derivate (z.B. Selenmethionin), Stilbene und deren Derivate (z.B. Stilbenoxid, Trans-Stilbenoxid), Superoxid-Dismutase und die erfindungsgemäß geeigneten Derivate (Salze, Ester, Ether, Zucker, Nukleotide, Nukleoside, Peptide und Lipide) dieser genannten Stoffe.

Besonders vorteilhaft im Sinne der vorliegenden Erfindung können wasserlösliche Antioxidantien eingesetzt werden.

Die Antioxidantien können die Haut und das Haar vor oxidativer Beanspruchung schützen. Bevorzugte Antioxidantien sind dabei Vitamin E und dessen Derivate sowie Vitamin A und dessen Derivate.

Die Menge des einen oder der mehreren Antioxidantien in den erfindungsgemäßen Zubereitungen beträgt vorzugsweise 0,001 bis 30 Gew.-%, besonders bevorzugt 0,05 bis 20 Gew.-% und insbesondere 1 bis 10 Gew.-%, bezogen auf das Gesamtgewicht der Zubereitungen.

Sofern Vitamin E und/oder dessen Derivate das oder die Antioxidantien darstellen, ist es vorteilhaft, deren jeweilige Konzentrationen aus dem Bereich von 0,001 bis 10 Gew.-%, bezogen auf das Gesamtgewicht der Zubereitung, zu wählen. Sofern Vitamin A, bzw. Vitamin-A-Derivate; bzw. Carotine bzw. deren Derivate das oder die Antioxidantien darstellen, ist es vorteilhaft, deren jeweilige Konzentrationen aus dem Bereich von 0,001 bis 10 Gew.-%, bezogen auf das Gesamtgewicht der Zubereitung, zu wählen.

In einer besonders bevorzugten Ausführungsform der Erfindung enthalten die kosmetischen oder pharmazeutischen Zubereitungen Antioxidantien ausgewählt aus Superoxid-Dismutase, Tocopherol (Vitamin E) und Ascorbinsäure (Vitamin C).

In einer weiteren bevorzugten Ausführungsform handelt es sich bei den erfindungsgemäßen Zubereitungen um Deodorantien und Antiperspirantien, die ein oder mehrere Substanzen ausgewählt aus antimikrobiell wirkenden Substanzen, Adstringentien und deodorierenden Stoffen enthalten. Diese Zubereitungen liegen vorzugsweise in Form von Sprays, Sticks, Pasten, Gelen oder Lotionen vor.

Bevorzugt geeignet als antimikrobielle Wirkstoffe sind Cetyttrimethylammoniumchlorid, Cetylpyridiniumchlorid, Benzethoniumchlorid, Diisobutylethoxyethyl-dimethylbenzylammoniumchlorid, Natrium N-Laurylsarcosinat, Natrium-N-Palmethylsarcosinat, Lauroylsarcosin, N-Myristoylglycin, Kalium-N-Laurylsarcosin, Trimethylammoniumchlorid, Natriumaluminiumchlorohydroxylactat, Triethylcitrat, Tricetylmethylammoniumchlorid, 2,4,4'-Trichloro-2'-hydroxydiphenylether (Triclosan), Phenoxyethanol, 1,5-Pentandiol, 1,6-Hexandiol, 3,4,4'-Trichlorocarbanilid (Triclocarban), Diaminoalkylamid, beispielsweise L-Lysinhexadecylamid, Citratschwermetallsalze, Salicylate, Piroctose, insbesondere Zinksalze, Pyrithione und deren Schwermetallsalze, insbesondere Zinkpyrithion, Zinkphenolsulfat, Farnesol und Kombinationen dieser Wirksubstanzen.

Die erfindungsgemäßen Zubereitungen enthalten die antimikrobiellen Wirkstoffe bevorzugt in Mengen bis 50 Gew.-%, besonders bevorzugt in Mengen von 0,01 bis 10 Gew.-% und insbesondere bevorzugt in Mengen von 0,1 bis 10 Gew.-%, bezogen auf die fertigen Zubereitungen.

Bevorzugte Adstringentien sind Oxide, bevorzugt Magnesiumoxid, Aluminiumoxid, Titandioxid, Zirkondioxid und Zinkoxid, Oxidhydrate, bevorzugt Aluminiumoxidhydrat (Böhmit) und Hydroxide, bevorzugt von Calcium, Magnesium, Aluminium, Titan, Zirkon oder Zink.

Die erfindungsgemäßen Zubereitungen enthalten die adstringenden Wirkstoffe bevorzugt in Mengen von 0 bis 50 Gew.-%, besonders bevorzugt in Mengen von 0,01 bis 10 Gew.-% und insbesondere bevorzugt in Mengen von 0,1 bis 10 Gew.-%, bezogen auf die fertigen Zubereitungen.

Als deodorierende Stoffe bevorzugt sind Allantoin und Bisabolol, Diese werden vorzugsweise in Mengen von 0,0001 bis 10 Gew.-% eingesetzt.

In einer weiteren bevorzugten Ausführungsform handelt es sich bei den erfindungsgemäßen Zubereitungen um Peelings. Diese liegen vorzugsweise in Form von Peeling-Cremes oder-gelen zum Reinigen und Glätten der Haut vor.

Die erfindungsgemäßen Zubereitungen können als weitere Hilfs- und Zusatzstoffe andere pulverförmige Stoffe, Füllkörper, kationische Polymere, Filmbildner, Verdickungs- und Dispergiermittel, Überfettungsmittel, feuchtigkeitsspendende Mittel, Stabilisatoren, biogene Wirkstoffe, Glycerin, Konservierungsmittel, Perlglanzmittel, Duftstoffe, Lösungsmittel, Trübungsmittel, weitere Wachse, ferner Eiweißderivate wie Gelatine, Collagenhydrolysate, Polypeptide auf natürlicher und synthetischer Basis, Eigelb, Lecithin, Lanolin und Lanolinderivate, Fettalkohole, Silicone, Erfrischungsmittel, beispielsweise Methylacetat, Stoffe mit keratolytischer und keratoplastischer Wirkung, Enzyme und Trägersubstanzen enthalten.

Des weiteren können als Füllkörper SiO₂, Silica, ZnO, Kaolin, mit SiO₂ modifiziertes Kaolin, Polytetrafluorethylen, Nylon, Talkum, Glimmer, Polymethylmethacrylat, Polyethylen, Polyether, Polycarbonate, Polyvinylchlorid, Polystyrol, Polyamide, Polyurethane, Polyacrylate, natürliche Polymere, Seidenpulver, mikrokristalline Cellulose, natürliche organische Verbindungen wie verkapselte oder unverkapselte Getreidestärke und Gemische davon eingesetzt werden.

An kationischen Polymeren stehen die unter der INCI-Bezeichnung "Polyquaternium" bekannten, insbesondere Polyquaternium-31, Polyquaternium-16, Polyquaternium-24, Polyquaternium-7, Polyquaternium-22, Polyquaternium-39, Polyquaternium-28, Polyquaternium-2, Polyquaternium-10, Polyquaternium-11, sowie Polyquaternium 37&mineral oil&PPG trideceth (Salcare SC95), PVP-dimethylaminoethylmethacrylat-Copolymer, Guar-hydroxypropyltriammoniumchloride, sowie Calciumalginat und Ammoniumalginat zur Verfügung. Ebenso geeignet sind kationische Cellulosederivate; kationische Stärke; Copolymere von Diallylammoniumsalzen und Acrylamiden; quaternierte Vinylpyrrolidon/Vinylimidazol-Polymere; Kondensationsprodukte von Polyglykolen und Aminen; quaternierte Kollagenpolypeptide; quaternierte Weizenpolypeptide; Polyethylenimine; kationische Siliconpolymere, wie z.B. Amidomethicone; Copolymere der Adipinsäure und Dimethylaminohydroxy-propyldiethylentriamin; Polyaminopolyamid und kationische Chitinderivate, wie beispielsweise Chitosan.

Geeignete Silikonverbindungen sind beispielsweise Dimethylpolysiloxan, Methylphenylpolysiloxane, cyclische Silikone und amino-, fettsäure-, alkohol-, polyether-, epoxy-, fluor- und/oder alkylmodifizierte Silikonverbindungen, sowie Polyalkylsiloxane, Polyalkylarylsiloxane, Polyethersiloxan-Copolymere, wie in US 5,104,645 und den darin zitierten Schriften beschrieben, die bei Raumtemperatur sowohl flüssig als auch harzförmig vorliegen können.

Geeignete Filmbildner sind, je nach Anwendungszweck Salze der Phenylbenzimidazolsulfonsäure, wasserlösliche Polyurethane, beispielsweise C₁₀-Polycarbamyl-Polyglycerolester, aber auch Polyvinylalkohol, Polyvinylpyrrolidon, sowie Copolymere, beispielsweise Vinylpyrrolidon/Vinylacetatcopolymer, wasserlösliche Acrylsäurepolymere/Copolymere bzw. deren Ester oder Salze, beispielsweise Partialestercopolymere der Acryl/Methacrylsäure und Polyethylenglykolether von Fettalkoholen, wie Acrylat/Steareth-20-Methacrylat Copolymer, wasserlösliche Cellulose, beispielsweise Hydroxymethylcellulose, Hydroxyethylcellulose, Hydroxypropylcellulose, wasserlösliche Quaterniums, Polyquaterniums, Carbocyvinyl-Polymere, wie Carbomere und deren Salze, Polysaccharide, beispielsweise Polydextrose und Glucan.

Als Überfettungsmittel können Substanzen wie beispielsweise Lanilin, Lecithin, polyethoxylierte Lanolinderivate, Lecithinderivate, Polyolfettsäureester, Monoglyceride und Fettsäurealkanolamide verwendet werden, wobei die letzteren gleichzeitig als Schaumstabilisatoren dienen.

Als feuchtigkeitsspendende Substanz stehen beispielsweise Isopropylpalmitat, Glycerin und/oder Sorbitol zu Verfügung, die bevorzugt in den Mengen von 0,1 bis 50 Gew.-% eingesetzt werden.

Als biogene Wirkstoffe können beispielsweise Pflanzenextrakte, beispielsweise Aloe Vera und Vitaminkomplexe, Bisabolol® , Allantoin® , Phytantriol® , Panthenol® , AHA-Säuren, Lokalanästhetika, Antibiotika, Antiphlogistika, Antiallergica, Corticosteoide, Sebostatika, Phanthenol, Allantoin und Proteine eingesetzt werden.

Als Konservierungsmittel eignen sich beispielsweise Phenoxyethanol, Diazolidinylharnstoff, Parabene, Pentandiol, butyliertes Hydroxytoluol, butyliertes Hydroxyanisol oder Sorbinsäure. Sie werden vorzugsweise in den Mengen von 0,001 bis 5 Gew.-%, besonders bevorzugt von 0,01 bis 3 Gew.-% und insbesondere bevorzugt von 0,1 bis 2 Gew.-%, bezogen auf die fertigen Zubereitungen, eingesetzt.

Als Farbstoffe können die für kosmetische und pharmazeutische Zwecke geeigneten und zugelassenen Substanzen verwendet werden.

Als perlglanzgebende Komponente bevorzugt geeignet sind Fettsäuremonoalkanolamide, Fettsäuredialkanolamide, Monoester oder Diester von Alkylenglykolen, insbesondere Ethylenglykol und/oder Propylenglykol oder dessen Oligomere, mit höheren Fettsäuren, wie z.B. Palmitinsäure, Stearinsäure und Behensäure, Monoester oder Polyester von Glycerin mit Carbonsäuren, Fettsäuren und deren Metallsalze, Ketosulfone oder Gemische der genannten Verbindungen. Besonders bevorzugt sind Ethylenglykoldistearate und/oder Polyethylenglykoldistearate mit durchschnittlich 3 Glykoleinheiten.

Sofern die erfindungsgemäßen Zubereitungen perlglanzgebende Verbindungen enthalten, sind diese bevorzugt in einer Menge von 0,1 bis 15 Gew.-% und besonders bevorzugt in einer Menge von 1 bis 10 Gew.-% in den erfindungsgemäßen Zubereitungen enthalten.

Als antifungizide Wirkstoffe eignen sich bevorzugt Ketoconazol, Oxiconazol, Bifonazole, Butoconazole, Cloconazole, Clotrimazole, Econazole, Enilconazole, Fenticonazole, Isoconazole; Miconazole, Sulconazole, Tioconazole Fluconazole, Itraconazole, Terconazole, Naftifine und Terbinafine, Zn-Pyrethion und Octopyrox.

Als Verdickungs- und Dispergiermittel besonders geeignet sind Ethylenglykolester von Fettsäuren mit 14 bis 22, besonders bevorzugt 16 bis 22, Kohlenstoffatomen, insbesondere Mono- und Di-ethylenglykolstearat. Ebenfalls bevorzugt geeignet sind Stearinmonoethanolamid, Stearindiethanolamid, Stearinisopropanolamid, Stearinmonoethanolamidstearat, Stearylstearat, Cetylpalmitat, Glycerylstearat, Stearamiddiethanolamiddistearat, Stearamidmonoethanolamidstearat, N,N-Dihydrocarbyl-(C₁₂-C₂₂)-amidobenzoesäure und deren lösliche Salze, N,N-Dihydrocarbyl-(C₁₆-C₁₈)-amidobenzoesäure und deren lösliche Salze und N,N-di(C₁₆-C₁₈)-amidobenzoesäure und deren Derivate. Weiterhin besonders geeignet sind Polyacrylate und Carbomere, insbesondere solche wasserlöslicher oder wasserquellbarer Copolymerisate auf Basis von Acrylamidoalkylsulfonsäuren und N-Vinylcarbonsäureamiden.

Zur Erhöhung der Farbintensität können die erfindungsgemäßen Zubereitungen die in kosmetischen Systemen üblichen Carrier enthalten, insbesondere Benzylalkohol, Vanillin (4-Hydroxy-3-methoxy-benzaldehyd), Isovanillin, p-Hydroxyanisol, 3-Hydroxy-4-methoxy-benzaldehyd, 2-Phenoxyethanol, Salicylaldehyd, 3,5-Dihydroxybenzaldehyd, 3,4-Dihydroxybenzaldehyd, 4-Hydroxyphenylacetamid, p-Hydroxybenzoesäure-methylester, p-Hydroxybenzaldehyd, m-Kresol, Hydrochinonmonomethylether, o-Fluorphenol, m-Fluorphenol, p-Fluorphenol, 2-(2'-Hydroxyphenoxy)-ethanol, 3,4-Methylendioxyphenol, Resorcinmonomethylether, 3,4-Dimethoxyphenol, 3-Trifluormethylphenol, Resorcinmonoacetat, Ethylvanillin, 2-Thiophenethanol, Milchsäurebutylester und Glykolsäurebutylester. Besonders vorteilhaft mit synergistischer Wirkung sind erfindungsgemäße Zubereitungen enthaltend Phenoxyethanol und/oder Benzylalkohol.

Als Solubilisatoren eignen sich prinzipiell alle ein- oder mehrwertigen Alkohole und ethoxylierten Alkohole. Bevorzugt werden Alkohole mit 1 bis 4 Kohlenstoffatomen, wie z.B. Ethanol, Propanol, Isopropanol, n-Butanol und Iso-Butanol, Glycerin und deren Mischungen eingesetzt. Weiterhin bevorzugt sind Polyethylenglykole mit einer relativen Molekülmasse unter 2000. Insbesondere bevorzugt sind Polyethylenglykole mit einer relativen Molekülmasse zwischen 200 und 600 in Mengen bis zu 45 Gew.-% und Polyethylenglykole mit einer relativen Molekülmasse zwischen 400 und 600 in Mengen von 0,5 bis 15 Gew.-%. Weitere geeignete Lösungsmittel sind beispielsweise Triacetin (Glycerintriacetat) und 1-Methoxy-2-propanol.

Als Stabilisatoren können Metallsalze von Fettsäuren, wie z.B. Magnesium-Aluminium- und/oder Zinkstearat eingesetzt werden.

Die erfindungsgemäßen Zubereitungen können mit konventionellen Ceramiden, Pseudoceramiden, Fettsäure-N-alkylpolyhydroxyalkylamiden, Cholesterin, Cholesterinfettsäureestern, Fettsäuren, Triglyceriden, Cerebrosiden, Phospholipiden und ähnlichen Stoffen als Pflegezusatz abgemischt werden.

Als Duft- bzw. Parfümöle können einzelne Riechstoffverbindungen, z.B. die synthetischen Produkte vom Typ der Ester, Ether, Aldehyde, Ketone, Alkohole und Kohlenwasserstoffe verwendet werden. Riechstoffverbindungen vom Typ der Ester sind z.B. Benzylacetat, Phenoxyethylisobutyrat, p-tert.-Butylcyclohexylacetat, Linalylacetat, Dimethylbenzylcarbinylacetat, Phenylethylacetat, Linalylbenzoat, Benzylformiat, Ethyl-methylphenylglycinat, Allylcyclohexylpropionat, Styrallylpropionät und Benzylsalicylat. Zu den Ethern zählen beispielsweise Benzytethylether, zu den Aldehyden z.B. die linearen Alkanale mit 8 bis 18C-Atomen, Citral, Citronellal, Citronellyloxyacetaldehyd, Hydroxycitronellal, Lilial und Bourgeonal, zu den Ketonen z.B. die lonone, alpha-Isomethylionon und Methylcedrylketon, zu den Alkoholen Anethol, Citronellol, Eugenol, Geranion, Linalool, Phenylethylalkohol und Terpineol, zu den Kohlenwasserstoffen gehören hauptsächlich die Terpene und Balsame. Bevorzugt werden Mischungen verschiedener Riechstoffe verwendet, die gemeinsam eine ansprechende Duftnote erzeugen.

Parfümöle können auch natürliche Riechstoffgemische enthalten, wie sie aus pflanzlichen oder tierischen Quellen zugänglich sind, z.B. Pinien-, Citrus-, Jasmin-, Lilien-, Rosen-, oder Ylang-Ylang-Öl. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl und Ladanumöl.

Weitere Zusatzstoffe können Silikonverbindungen sein, vorzugsweise Dimethylpolysiloxan, Methyl-phenylpolysiloxane, cyclische Silikone sowie amino-, fettsäure-, alkohol-, polyether-, epoyx-, fluor- und/oder alkylmodifizierte Silikonverbindungen, beispielsweise Alkylsilicone SilCare® Silicone 41M10, SilCare® Silicone 41 M15, SilCare® Silicone 41 M20, SilCare® Silicone 41 M30 (Clariant), Alkyltrimethicone SilCare® 31 M30, SilCare® 31 M40, SilCare® 31 M 50, SilCare® 31 M 60 (Clariant), Phenyltrimethicone SilCare® 15M30, SilCare® 15M40, SilCare® 15M50, SilCare® 15M60 (Clariant), Polyalkylarylsiloxane und Polyethersiloxan-Copolymere.

Die erfindungsgemäßen Zubereitungen können die oben genannten Silikonverbindungen vorzugsweise in den Gewichtsmengen von 0,1 bis 20 Gew.-%, besonders bevorzugt von 0,2 bis 15 Gew.-% und insbesondere bevorzugt von 0,5 bis 10 Gew.-% bezogen auf die fertigen Zubereitungen enthalten.

Die Zubereitungen besitzen üblicherweise einen pH-Wert im Bereich von 2 bis 12 und bevorzugt in einem Bereich von 3 bis 8.

Eine herausragende Eigenschaft der erfindungsgemäß eingesetzten Copolymerwachse sind deren Verdickungsvermögen von Ölen.

Die nachfolgenden Beispiele und Anwendungen sollen die Erfindung näher erläutern, ohne sie jedoch darauf zu beschränken (bei allen Prozentangaben handelt es sich um Gewichtsprozent).

### Beispiel A: Copolymerwachs 1

Copolymer aus C₃₀₊-α-Olefinen mit Maleinsäureanhydrid

### Ansatz:

| | |
|---|---|
| C₃₀₊-α-Olefin | 1680,00 g (3,00 mol) |
| Maleinsäureanhydrid | 309,02 g (3,15 mol) |
| di-tert.-Butylperoxid | 15,45 g (5 Gew.-% bezogen auf Maleinsäureanhydrid) |
| Synperonic LFRA 40 | 30,90 g (10 Gew.-%, bezogen auf Maleinsäureanhydrid) |

### Herstellung:

Das Olefin wird bei 100°C aufgeschmolzen, der Ansatz auf 160°C erhitzt, Synperonic LFRA 40 zugegeben und dann portionsweise alle 15 Minuten di-tert.-Butyiperoxid. Nach der ersten Peroxid-Zugabe wird zusätzlich Maleinsäureanhydrid in Portionen alle 15 Minuten unter Rückfluss zudosiert und es wird 3 Stunden nachgerührt. Die freien Monomere werden 1 Stunde im Vakuum entfernt.

Säurezahl: 145

### Beispiel C: Copolymerwachs 3

Copolymer aus C_{26/28}-α-Olefinen mit Maleinsäureanhydrid

### Ansatz:

| | |
|---|---|
| C_{26/28}-α-Olefin | 1196,80 g (3,20 mol) |
| Maleinsäureanhydrid | 329,62 g (3,36 mol) |
| di-tert.-Butylperoxid | 16,48 g (5 Gew.-% bezogen auf Maleinsäureanhydrid) |
| Synperonic LFRA 40 | 32,96 g (10 Gew.-%, bezogen auf Maleinsäureanhydrid) |

### Herstellung:

Das Olefin wird bei 100°C aufgeschmolzen, der Ansatz auf 160°C erhitzt, Synperonic LFRA 40 zugegeben und dann portionsweise alle 15 Minuten di-tert.-Butylperoxid. Nach der ersten Peroxid-Zugabe wird zusätzlich Maleinsäureanhydrid in Portionen alle 15 Minuten unter Rückfluss zudosiert und es wird 3 Stunden nachgerührt. Die freien Monomere werden 1 Stunde im Vakuum entfernt.

### Säurezahl: 166,0

Chemische Bezeichnung der eingesetzten Handelsprodukte:

Synperonic LFRA 40: Fettalkoholethoxylat/-propoxylat

### Meßmethoden:

Säurezahl mg KOH/g ISO 2114

### Formulierungsbeispiele

### Beispiel 1: W/O-Creme

| | | | |
|---|---|---|---|
| A | Hostacerin® DGI | | 4,00 % |
| | Copolymerwachs 3 | Clariant | 5,00 % |
| | Magnesiumstearat | | 1,00% |
| | Mineralöl, niedrigviskos | | 5,00 % |
| | Vaseline | | 10,00 % |
| | Cetiol® V | | 5,00 % |
| | | | |
| B | 1,2-Propylenglycol | | 3,00 % |
| | Wasser | | ad 100 % |
| | Konservierungsmittel | | q.s. |
| C | Duftstoff | | 0,40 % |

### Herstellung:

- I: Schmelzen von A bei 80°C
- II: Erwärmen von B auf 80°C
- III: Einrühren von II in I
- IV: Rühren bis Temperatur von 35°C erreicht ist
- V: Zugabe von C zu IV bei 35°C

### Beispiel 2: O/W-Creme

| | | | |
|---|---|---|---|
| A | Hostacerin® DGI | | 2,00 % |
| | SilCare® Silicone 31 M30 | Clariant | 4,00 % |
| | Perliquidum | | 4,00 % |
| | Copolymerwachs 1 | Clariant | 2,00 % |
| | Eutanol G | Clariant | 4,00 % |
| | Isopropylpalmitat | Clariant | 4,00 % |
| | Carbopol 980 | | 0,70 % |
| | | | |
| B | Hostapon® KCG | | 0,60 % |
| | Natronlauge (10% in Wasser) | | 2,10 % |
| | Konservierungsmittel | | q.s. |
| | Duftstoff | | 0,40 % |
| | dest. Wasser | | ad 100 % |

### Herstellung:

- I: Erwärmen von A auf 80°C
- II: Erwärmen von B auf 80°C
- III: Emulgieren durch langsames Einrühren von B in A.

### Beispiel 3: Haarkur

| | | |
|---|---|---|
| A | Wasser | ad 100 % |
| | | |
| B | Genamin® KSL | 7 % |
| | Hostaphat® KL 340 D | 1,5 % |
| | Copolymerwachs 1 | 1 % |
| | Genapol® PDB | 4 % |
| | Jojobaöl | 1 % |
| | Propylenglykol | 0,8 % |
| | Isopropylpalmitat | 1% |
| | Dow Corning® 190 | 0,8% |
| | Extrapon | 0,3% |
| | Vitamin E | 0,3 % |
| | Panthenol (Vitamin B 5) | 0,5 % |
| | | |
| C | Zitronensäure | 0,2 % |

### Herstellung:

- I: A auf 75°C erwärmen
- II: B auf 75°C erwärmen
- III: A unter Rühren zu B geben und Kaltrühren
- IV: mit C auf pH 6 einstellen

### Beispiel 4: Cremespülung

| | | |
|---|---|---|
| A | Tylose® H 100 000 YP2 | 1,5 % |
| | Wasser dest. | ad 100 % |
| | | |
| B | Hostaphat® KL 340 D | 1,5 % |
| | Genapol® PDB | 4% |
| | Copolymerwachs 1 | 1 % |
| | | |
| C | Zitronensäure | 0,2 % |

### Herstellung:

- I: Tylose im Wasser bei Raumtemperatur unter Rühren aufquellen
- II: I auf 75°C erwärmen
- III: B auf 75°C erwärmen
- IV: II zu III geben und kaltrühren
- V: mit C auf pH 6 einstellen

### Beispiel 5: Antiperspirant

| | | | |
|---|---|---|---|
| A | Locron® L | Clariant | 10,00 % |
| | Ethanol | | 50,00 % |
| | Farnesol | | 0,50 % |
| | Duftstoff | | 0,20 % |
| | Copolymerwachs 1 | | 0,50 % |
| | Wasser dest. | | ad 100 % |
| | Extrapon Avocado special | | 1,50 % |

### Herstellung:

Mischen der Komponenten A

### Beispiel 6: W/O-Antiperspirant Creme

| | | |
|---|---|---|
| A | Abil EM90 | 2,0 % |
| | Abil B8839 | 20,0 % |
| | Copolymerwachs 3 | 2,0 % |
| | | |
| B | Aloxicoll L | 17,0 |
| | Wasser dest. | ad 100 % |
| | Parfümöl | q.s. |
| | Konservierungsmittel | q.s. |

### Herstellung:

- I: Phase B langsam unter Rühren bei Raumtemperatur zur Phase A geben.
- II: Homogenisieren

### Beispiel 7: Klarer Deo-Stick

| | | | |
|---|---|---|---|
| A | OCTOPIROX® | (Clariant) | 0,10 % |
| | Copolymerwachs 3 | | 3 % |
| | Propylene Glycol | | 71,00 % |
| | Rewoderm 66E | | 5,00 % |
| | Natriumstearat | | 5,00% |
| | GENAPOL® HS 020 | (Clariant) | 1,00% |
| | Wasser | | ad 100 % |

### Herstellung:

- I: Mischen der Komponenten A bei ca. 50°C und rühren bis die Lösung klar ist
- II: Abfüllen und Abkühlen

### Beispiel 8: Alkoholfreier Deodorant-Roll-on (opak)

| | | |
|---|---|---|
| A | Tegodeo CW 90 | 2,0 % |
| | Polyethylenglycol(3)laurylether | 1,0 % |
| | Triethanolamin | 1,0 % |
| | | |
| B | Copolymerwachs 1 | 1,2 % |
| | Wasser dest. | ad 100 % |
| | | |
| C | Tagat R 40 | 3,0 % |
| | Parfümöl | q.s. |
| | Konservierungsmittel | q.s. |
| | | |
| D | Zitronensäure (50 %ig in Wasser) | 0,2 % |

### Herstellung:

- I: Phasen A und B separat auf 80°C erwärmen
- II: Phase B in Phase A einrühren und homogenisieren
- III: Unter langsamem Rühren abkühlen
- IV: Bei 30°C Phase C hinzufügen
- V: Den pH-Wert mit Hilfe von Phase D einstellen

### Beispiel 9: Sonnenschutz-Gel

| | | | |
|---|---|---|---|
| A | Crodamol® AB | | ad 100% |
| | Neo Heliopan® AV | | 7,50 % |
| | SilCare® Silicone 41M80 | (Clariant) | 5,00 % |
| | Copolymerwachs 3 | | 1 % |
| | | | |
| B | Neo Heliopan® BB | | 3,00 % |

### Herstellung:

- I: A auf ca. 80°C erwärmen
- II: B in A lösen
- III: Abfüllen und auf 25°C abkühlen ohne rühren

### Beispiel 10: Grundierung

| | | | |
|---|---|---|---|
| A | Nexbase® 2004 FG | | 9,00 % |
| | Myritol® 318 | | 5,00 % |
| | Almond Oil | | 4,00 % |
| | SilCare® Silicone 31 M40 | (Clariant) | 4,00 % |
| | SilCare® Silicone 41 M15 | (Clariant) | 3,00% |
| | Genapol® HS020 | (Clariant) | 1,60 % |
| | Genapol® HS200 | (Clariant) | 2,40 % |
| | Copolymerwachs 1 | | 2,00 % |
| | | | |
| B | Vanclay® | | 1,50% |
| | Talc | | 3,00% |
| | Iron Oxide Pigments | | 7,90 % |
| | | | |
| C | Glycerin | | 5,00 % |
| | Wasser | | ad 100 % |
| | Aristoflex® AVC | (Clariant) | 0,40% |
| | | | |
| D | Duftstoff | | q.s. |
| | Nipaguard® PDU | (Clariant) | q.s. |

### Herstellung:

- I: Mischen und schmelzen der Komponenten A bei ca. 70°C
- II: Zugabe von B zu I bei ca. 70°C unter Rühren
- III: Mischen von C bis Aristoflex® AVC gelöst ist und Erwärmen auf ca. 70°C
- IV: Zugabe von C zu II unter Rühren und Homogenisieren.
- V: Zugabe von D zu IV bei < 40°C

### Beispiel 11: Mascara

| | | | |
|---|---|---|---|
| A | Tylose® H 4000 G4 | | 0,70% |
| | 1,2-Propylengtykol | | 1,00 % |
| | Wasser | | ad 100 % |
| | | | |
| B | Triethanolamine 99% | | 1,20 % |
| | | | |
| C | Stearinsäure | | 3,00 % |
| | SilCare® Silicone 41M15 | (Clariant) | 1,00 % |
| | SilCare® Silicone 31M40 | (Clariant) | 2,00 % |
| | Tegocare® 450 | | 4,00 % |
| | Nexbase® 2006 | | 2,00 % |
| | Bienenwachse | | 2;50 % |
| | Candelilla Wachs | | 2,50 % |
| | Copolymerwachs 3 | | 3,50 % |
| | | | |
| D | Pigmente | | 10,00 % |
| | | | |
| E | Nipagin® M | (Clariant) | 0,20 % |
| | Nipasol® M | (Clariant) | 0,10 % |
| | | | |
| F | Duftstoff | | q.s |

### Herstellung:

- I: Komponenten A bei Raumtemperatur unter Rühren aufquellen; auf 85°C erwärmen
- II: Zugabe von B zu A und rühren
- III: Schmelzen der Komponenten C auf ca 85°C
- IV: Zugabe von D zu III unter Rühren bei 85°C
- V: Zugabe von II zu IV unter kräftigem Rühren (ca 15 Minuten bei 85°, weitere 15 Minuten ohne Erwärmen)
- VI: Zugabe von E und F zu V bei ca 35 bis 40°C
- VII: Abfüllen bei 35°C bis 40°C.

### Beispiel 12: Tensidfreie Lotion mit erfrischender, belebender Wirkung

| | | |
|---|---|---|
| A | Jojoba oil | 2,00 % |
| | Almondöl | 3,00 % |
| | Cetiol® V | 3,00 % |
| | | |
| B | Copolymerwachs 1 | 2,00 % |
| | | |
| C | Glycerin | 3,00% |
| | Menthol | 0,70 % |
| | Campher | 0,30 % |
| | Ethanol | 5,00 % |
| | Wasser | ad 100 % |
| | Konservierungsmittel | q.s. |
| | | |
| D | Duftstoff | 0,30% |
| | | |
| E | Zitronensäure (50 %ig in Wasser) | q.s. |

### Herstellung:

- I: A und B mischen
- II: Lösung von C in I einrühren
- III: D zu II geben
- IV: homogenisieren
- V: pH-Wert mit Hilfe von Phase E auf 6,00 einstellen.

### INCI Bezeichnung der eingesetzten Handelsprodukte:

| | | |
|---|---|---|
| Abil B8839 | | Cyclopentasiloxan/ Cyclohexasilöxan |
| Abil EM90 | | Cetyldimethicon/Copolyol |
| Aloxicoll L. | | Alumiunium Chlorohydrat |
| Aristoflex® AVC | (Clariant) | Ammonium Acyloyldimethyltaurate/VP Copolymer |
| Carbopol 980 | | Polyacrylat |
| Cetiol® V | (Cognis) | Decyloleat |
| Crodamol® AB | | C₁₂₋₁₅ Alkyl Benzoate |
| Dow Corning® 190 | (Dow Corning) | Dimethicon Copolyol |
| Euperlan® PK 3000 | (Henkel) | Glycol Distearat, Laureth-4, Cocamidopropyl Betain |
| Eutanol G | | 2-Octyldodecanol |
| Extrapon® | (Dragoco) | Pflanzliche Extrakte |
| Extrapon Avocado special | | Wasser/ |
| Ethoxydiglycol/Propylenglycol/ | | Butylenglycol/Persea Gratissima |
| Extrakt | | |
| Genamin® CTAC | (Clariant) | Cetyltrimethylammonium Chlorid |
| Genamin® KDM-P | (Clariant) | Behenyltrimethylammonium Chlorid |
| Genamin® KSL | (Clariant) | PEG- 5 Stearyl Ammonium Lactat |
| Genamin® STAC | (Clariant) | Stearytrimethylammonium Chlorid |
| GENAPOL® HS 020 | (Clariant) | Steareth-2 |
| Genapol® HS200 | (Clariant) | Steareth-20 |
| Genapol® PDB | (Clariant) | Glycol Distearat, Laureth-4, Cocamidopropyl Betain |
| Hostacerin® DGI | | Polyglyceryl-2-Sesquüsostearat |
| Hostacerin® T- 3 | | Ceteareth-3 |
| Höstaphat® KL 340 D | | Trilaureth-4 Phosphat |
| Hostapon® KCG | | Natriumcocoylglutamat |
| Locron® L | (Clariant) | Aluminium Chlorohydrat |
| Myrito^{l}® 318 | | Capric/Caprylic Triglyceride |
| Neo Heliopan® AV | | Ethylhexyl Methoxycinnamate |
| Neo Heliopan® BB | | Benzophenone-3 |
| Nexbase® 2004 FG | | Hydrogenated Poly-1-Decene |
| Nexbase® 2006 | | Poly-1-Decen. |
| Nipagin® M | (Clariant) | Methylparaben |
| NIPAGUARD® CMB | (Clariant) | Triethyfenglycol/ Benzylalkohol/Propylenglycol/ |
| Nipaguard® PDU | (Clariant) | Propylene Glycol (and) Diazolidinyl |
| Urea | | (and) Methylparaben (and) Propylparaben |
| Nipasol® M | (Clariant) | Propylparaben |
| OCTOPIROX® | (Clariant) | Piroctone Olamine |
| Perliquidum | | Paraffinöl |
| Rewoderm 66E Isostearate | | |
| SilCare® Silicone 31 M40 | (Clariant) | Caprylyl Trimethicone |
| SilCare® Silicone 31 M50 | (Clariant) | Caprylyltrimethicon |
| SilCare® Silicone 41 M15 | (Clariant) | Caprylylmethicon |
| SilCare® Silicone 41 M80 | (Clariant) | C₂₄₋₂₈ Alkyl Dimethicone |
| Tagat R40 | | PEG-40 hydrogeniertes Castoröl |
| Tegocare® 450 | | Polyglyceryl-3 Methylglucose Distearat |
| Tegodeo CW 90 | | Zinkricinoleat/ Tetrahydroxypropyl-Ethylendiamin/Laureth-3/Propylenglycol |
| Tylose® H 100000 YP2 | | Hydroxyethylcellulose |
| Tylose® H 4000 G4® | | Hydroxyethylcellulose |
| Vanclay® | | Kaolin |

## Patentansprüche

1. Kosmetische, pharmazeutische oder dermatologische Zubereitung, **dadurch gekennzeichnet, dass** sie ein oder mehrere Copolymerwachse, enthaltend
a) eine oder mehrere Struktureinheiten -CH₂-CHR-, worin R für eine lineare oder verzweigte Alkylgruppe mit 24 bis 58 Kohlenstoffatomen steht,
b) eine oder mehrere Struktureinheiten der Formeln (I) - (IV) 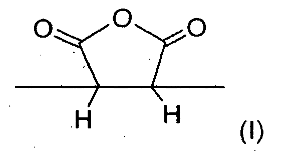 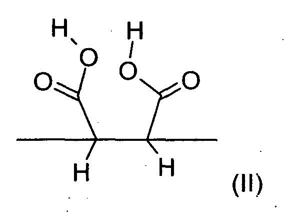 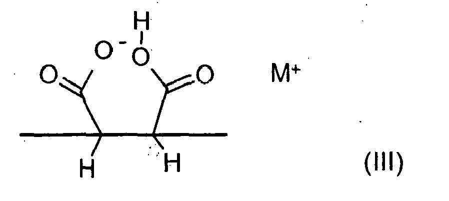 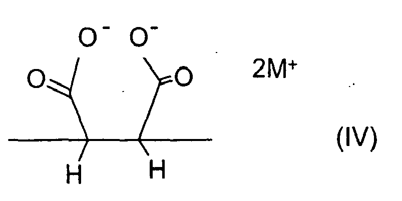 worin M⁺ für Li⁺, Na⁺, K⁺, Mg⁺⁺/2, Ca⁺⁺/2, Al⁺⁺⁺/3, NH₄⁺, Monoalkylammonium-, Dialkylammonium-, Trialkylammonium- und/oder Tetraalkylammoniumionen, wobei es sich bei den Alkylsubstituenten dieser Ammoniumionen jeweils unabhängig voneinander um (C₁-C₂₂)-Alkylreste oder (C₂-C₁₀)-Hydroxyalkylreste handeln kann, die auch ethoxyliert sein können und wobei die 1 bis 4 Ethylenoxid-haltigen Reste dieser Ammoniumionen jeweils unabhängig voneinander von 2 bis 10 Ethylenoxideinheiten enthalten können und den gleichen oder unterschiedliche Ethoxylierungsgrade aufweisen können, steht, und
c) gegebenenfalls eine oder mehrere Struktureinheiten der Formeln 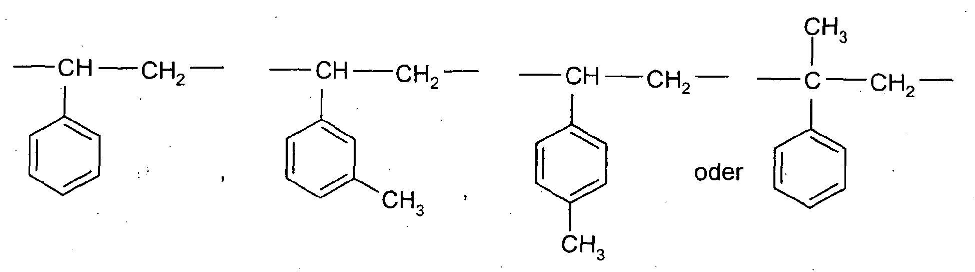 mit der Maßgabe, daß die Struktureinheiten der Komponenten a), b) und gegebenenfalls c) im Wesentlichen alternierend angeordnet sind und die Anzahl der Struktureinheiten der Komponente a), aller Struktureinheiten der Komonente b) zusammen und gegebenenfalls aller Struktureinheiten der Komponente c) zusammen jeweils im Bereich von 3 bis 60 liegt, enthält.

2. Zubereitung nach Anspruch 1, **dadurch gekennzeichnet, dass** die Copolymerwachse Struktureinheiten der Komponente a) enthalten, worin R für eine lineare oder verzweigte Alkylgruppe mit 24 und/oder 26 Kohlenstoffatomen steht.

3. Zubereitung nach Anspruch 1, **dadurch gekennzeichnet, dass** die Copolymerwachse Struktureinheiten der Komponente a) enthalten, worin R für eine lineare oder verzweigte Alkylgruppe mit 28 bis 58 Kohlenstoffatomen steht.

4. Zubereitung nach einem oder mehreren der Ansprüche 1 bis 3, **dadurch gekennzeichnet, dass** die Copolymerwachse Struktureinheiten der Komponente a) und Struktureinheiten der Komponente b) gemäß Formel (I) enthalten.

5. Zubereitung nach einem oder mehreren der Ansprüche 1 bis 4, **dadurch gekennzeichnet, dass** die Copolymerwachse Struktureinheiten der Komponente a) und Struktureinheiten der Komponente b) gemäß Formel (II) enthalten.

6. Zubereitung nach einem oder mehreren der Ansprüche 1 bis 5, **dadurch gekennzeichnet, dass** die Copolymerwachse Struktureinheiten der Komponente a) und eine oder mehrere Struktureinheiten der Komponente b) ausgewählt aus Formel (III) und Formel (IV) enthalten.

7. Zubereitung nach einem oder mehreren der Ansprüche 1 bis 6, **dadurch gekennzeichnet, dass** die Copolymerwachse die Komponenten a) und b) in den molaren Verhältnissen Komponente a) : Komponente b) von 1:1 - 2, vorzugsweise von 1:1-1,1 und besonders bevorzugt von 1:1,05 enthalten.

8. Zubereitung nach einem oder mehreren der Ansprüche 1 bis 7, **dadurch gekennzeichnet, dass** die Copolymerwachse Molekulargewichte im Bereich von 1300 bis 50 000, vorzugsweise im Bereich von 5000 bis 30 000 und besonders bevorzugt im Bereich von 10 000 bis 20 000 haben.

9. Zubereitung nach einem oder mehreren der Ansprüche 1 bis 8, **dadurch gekennzeichnet, dass** es sich um einen Abdeckstift, Akne-Stift, Lippenstift, ein Make-up, eine Grundierung, ein Gesichtspuder, Rouge, eine Mascara, einen Lidschatten, Eye-liner, eine Peeling-Creme, ein Haarwachs, Haar-Stylingmittel, Styling-Fluid, einen Haarschaum, ein Haargel, Haarspray, eine Mousse, ein Haaröl, ein Spitzenfluid, eine Haarkur, Nachtcreme, Pflegecreme, Nährcreme, Parfumcreme, Bodylotion, Salbe, ein Lippenpflegemittel, Sonnenschutzmittel, Deodorant, Antiperspirant, coloriertes Gel in Form eines Stifts wie z.B. eines Mehrphasenstifts, einen Stick, eine Paste, ein Puder, eine Creme, einen Cremeschaum, eine Lotion, eine selbstschäumende, schaumförmige, nachschäumende oder schäumbare Emulsion, ein Gel, Roll-On-Präparat oder einen Schaum handelt.

10. Zubereitung nach einem oder mehreren der Ansprüche 1 bis 9, **dadurch gekennzeichnet, dass** sie in Form einer Emulsion vorliegt.

11. Zubereitung nach Anspruch 10, **dadurch gekennzeichnet, dass** sie in Form einer Öl-in-Wasser-Emulsion vorliegt.

12. Zubereitung nach Anspruch 11, **dadurch gekennzeichnet, dass** sie als Emulsion vom Typ Öl-in-Wasser, vorzugsweise als kosmetische oder dermatologische Emulsion vom Typ Öl-in-Wasser, vorliegt und bezogen auf das Gesamtgewicht der Zubereitung
a) bis zu 95 Gew.-%, vorzugsweise 60 bis 92 Gew.-%, besonders bevorzugt 70 bis 90 Gew.-%, insbesondere bevorzugt 75 bis 85 Gew.-% einer Wasserphase,
b) bis zu 40 Gew.-%, vorzugsweise 1 bis 40 Gew.-%, besonders bevorzugt 2 bis 25 Gew.-%, insbesondere bevorzugt 5 bis 20 Gew.-% einer Ölphase,
c) bis zu 15 Gew.-%, vorzugsweise 0,5 bis 12 Gew.-%, besonders bevorzugt 1 bis 8 Gew.-%, insbesondere bevorzugt 1 bis 5 Gew.-% eines oder mehrerer Emulgatoren und
d) bis zu 5 Gew.-%, vorzugsweise 0,01 bis 5 Gew.-%, besonders bevorzugt 0,05 bis 3 Gew.-%, insbesondere bevorzugt 0,1 bis 2 Gew.-% an Copolymerwachs enthält.

13. Zubereitung nach Anspruch 11, **dadurch gekennzeichnet, dass** sie als Gelcreme vom Typ Öl-in-Wasser, vorzugsweise als kosmetische oder dermatologische Gelcreme vom Typ Öl-in-Wasser, vorliegt und bezogen auf das Gesamtgewicht der Zubereitung
a) bis zu 95 Gew.-%, vorzugsweise 50 bis 95 Gew.-%, besonders bevorzugt 70 bis 90 Gew.-%, insbesondere bevorzugt 75 bis 85 Gew.-% einer Wasserphase,
b) bis zu 30 Gew.-%, vorzugsweise 1 bis 30 Gew.-%, besonders bevorzugt 3 bis 25 Gew.-%, insbesondere bevorzugt 5 bis 15 Gew.-% einer Ölphase,
c) bis zu 5 Gew.-%, vorzugsweise 0,5 bis 5 Gew.-%, besonders bevorzugt 0,2 bis 4 Gew.-%, insbesondere bevorzugt 0,5 bis 3 Gew.-% eines oder mehrerer Emulgatoren und
d) bis zu 5 Gew.-%, vorzugsweise 0,01 bis 5 Gew.-%, besonders bevorzugt 0,05 bis 3 Gew.-%, insbesondere bevorzugt 0,1 bis 2 Gew.-% an Copolymerwachs enthält.

14. Zubereitung nach Anspruch 10, **dadurch gekennzeichnet, dass** sie in Form einer Wasser-in-Öl-Emulsion vorliegt.

15. Zubereitung nach Anspruch 14, **dadurch gekennzeichnet, dass** sie als Emulsion vom Typ Wasser-in-Öl, vorzugsweise als kosmetische oder dermatologische Emulsion vom Typ Wasser-in-Öl, vorliegt und bezogen auf das Gesamtgewicht der Zubereitung
a) bis zu 95 Gew.-%, vorzugsweise 40 bis 95 Gew.-%, besonders bevorzugt 50 bis 90 Gew.-%, insbesondere bevorzugt 60 bis 85 Gew.-% einer Wasserphase,
b) bis zu 60 Gew.-%, vorzugsweise 2 bis 60 Gew.-%, besonders bevorzugt 5 bis 40 Gew.-%, insbesondere bevorzugt 10 bis 30 Gew.-% einer Ölphase,
c) bis zu 20 Gew.-%, vorzugsweise 0,5 bis 20 Gew.7%, besonders bevorzugt 1 bis 15 Gew.-%, insbesondere bevorzugt 4 bis 12 Gew.-% eines oder mehrerer Emulgatoren und
d) bis zu 5 Gew.-%, vorzugsweise 0,01 bis 5 Gew.-%, besonders bevorzugt 0,05 bis 3 Gew.-%, insbesondere bevorzugt 0,1 bis 2 Gew.-% an Copolymerwachs enthält.

16. Zubereitung nach einem oder mehreren der Ansprüche 1 bis 15, **dadurch gekennzeichnet, dass** die Copolymerwachse in mikronisierter Form eingesetzt werden.

17. Zubereitung nach einem oder mehreren der Ansprüche 1 bis 16, **dadurch gekennzeichnet, dass** sie in Form einer Dispersion vorliegt und
a) ein Trägermaterial, vorzugsweise ein oder mehrere Ölkomponenten und/oder Lösungsmittel,
b) einen oder mehrere Emulgatoren und
c) neben den in Anspruch 1 genannten Copolymerwachsen gegebenenfalls ein oder mehrere weitere Wachse
enthält.

18. Zubereitung nach einem oder mehreren der Ansprüche 1 bis 17, **dadurch gekennzeichnet, dass** sie als dekoratives Mittel vorliegt.

19. Zubereitung nach einem oder mehreren der Ansprüche 1 bis 18, vorzugsweise in Form eines Puders, Preßlings, einer Paste, Creme oder eines Sticks, **dadurch gekennzeichnet, dass** sie ein oder mehrere Farbmittel, vorzugsweise ausgewählt aus Farblacken, Toner und Pigmenten, enthält.

20. Zubereitung nach einem oder mehreren der Ansprüche bis 19, **dadurch gekennzeichnet, dass** sie einen oder mehrere UV-Lichtschutzfilter enthält, vorzugsweise ein Sonnenschutzmittel ist, und besonders bevorzugt in Form eines Sprays, Sticks, einer Paste, eines Gels oder einer Lotion vorliegt.

21. Zubereitung nach einem oder mehreren der Ansprüche 1 bis 20, **dadurch gekennzeichnet, dass** sie ein oder mehrere Antioxidantien enthält.

22. Zubereitung nach einem oder mehreren der Ansprüche 1 bis 17 und 19 bis 21, vorzugsweise in der Form eines Sprays, Sticks, einer Paste, eines Gels oder einer Lotion, **dadurch gekennzeichnet, dass** sie ein Deodorant oder Antiperspirant ist und ein oder mehrere Substanzen ausgewählt aus antimikrobiell wirkenden Substanzen, Adstringentien und deodorierenden Stoffen enthält.

23. Zubereitung nach Anspruch 22, **dadurch gekennzeichnet, dass** sie ein oder mehrere deodorierende Stoffe enthält und diese ausgewählt sind aus Allantoin und Bisabolol.

24. Zubereitung nach einem oder mehreren der Ansprüche 1 bis 17 und 19 bis 21, **dadurch gekennzeichnet, dass** sie ein Peeling ist.
